# EUROPEAN PATENT SPECIFICATION

(11) **EP 0 511 003 B1**
(45) Date of publication and mention of the grant of the patent: **24.03.1999**
(21) Application number: 92303717.0
(22) Date of filing: 24.04.1992
(51) Int. Cl.: C07K 14/60, A61K 38/25

(54) **Superactive GRF analogs**
Superaktive Analoga des Wachstumshormon-Releasingfaktors
Analogues superactifs du facteur libérant l'hormone de croissance

(30) Priority: 26.04.1991 US 692090
(43) Date of publication of application: 28.10.1992
(73) Proprietor: ELI LILLY AND COMPANY, Indianapolis, Indiana 46285 (US)
(72) Inventor: Dimarchi, Richard Dennis, Carmel, IN 46032 (US); Heiman, Mark Louis, Indianapolis, IN 46250 (US); Shields, James Edwin, Noblesville, IN 46060 (US); Smiley, David Lee, Greenfield, IN 46140 (US)
(74) Representative: Tapping, Kenneth George

(56) References cited:
- EP-A- 188 214
- US-A- 4 528 190
- US-A- 4 626 523
- US-A- 4 628 043
- CHEMICAL ABSTRACTS, vol. 106, no. 3, January 19, 1987, Columbus, Ohio, USA; DAVID H. COY et al. "Differential effects of N-terminal modifications on the biological potencies of growth hormone releasing factor analogs with varying chain lengths" page 92, column 1, abstract-no. 13 063e
- CHEMICAL ABSTRACTS, vol. 111, no. 5, July 31, 1989, Columbus, Ohio, USA; KAZUKI SATO et al. "Structure-activity relations of growth hormone-releasing factor (GRF)" page 80, column 1, abstract-no. 33 758s
- CHEMICAL ABSTRACTS, vol. 109, no. 17, October 24, 1988, Columbus, Ohio, USA; MASAHIRO IWAKURA et al. "Expression of chemically synthesized genes in Escherichia coli" page 210, column 2, abstract-no. 143 885x

## Description

Growth hormone is a secretory protein of the pituitary gland of animals having wide ranging developmental effects on the organism. Artificial manipulation of growth hormone levels has been demonstrated to have significant therapeutic utility. Human growth hormone supplementation has been shown to be an effective treatment for growth hormone deficiencies and their related disease states in humans. Apart from this application, studies have uncovered new and significant properties of growth hormone which lend further importance to the ability to control growth hormone levels. For example, recent clinical studies indicate that growth hormone supplementation may be useful in combatting the maladies of aging in humans. Elevated growth hormone levels in animals have been shown to result in increased lean muscle mass. One application of this latter observation could result in higher production of leaner meat products or in the production of larger and/or stronger animals.

While growth hormone is naturally produced by the pituitary gland, the secretion of growth hormone into the bloodstream is controlled by a second protein, Growth Hormone Releasing Factor (GRF). This hormone is also commonly known in the art as somatocrinin, Growth Hormone Releasing Hormone (GHRH), and Growth Releasing Hormone (GRH). Consequently there are two ways to approach the problem of increasing circulating levels of growth hormone: (1) increase the level of human growth hormone in the organism directly or (2) increase the organism's natural tendency to produce growth hormone. The latter strategy may be achieved via supplementation with GRF. GRF has been demonstrated to increase the circulatory levels of growth hormone in vivo. Rivier, et al. (1982) Nature 300:276. The effect of GRF (and various structural analogs thereof) on growth hormone production has been widely studied. A primary obstacle to the use of GRF as a direct supplement is its short lifespan in vivo. Frohman, L.A., et al. (1986) J. Clin. Invest. 78 906. More potent and/or longer lasting GRF molecules are therefore desirable for the development of effective human therapeutic or animal husbandry agents.

The structure of GRF has been modified in numerous ways resulting in longer lasting and/or more potent GRF analogs. It has been demonstrated that the first 29 amino acids from the N-terminus are sufficient to retain full GRF activity. Speiss, et al. (1982) Biochemistry 21, 6037. One strategy has been the incorporation of novel D-amino acid residues in various regions of the GRF molecule. Lance, V.A., et al. (1984) Biochem. Biophys.Res.Commun. 119 265; Coy, D.H., et al. (1986) Peptides 8(suppl. 1), 49. Another strategy has modified the peptide backbone of GRF by the incorporation of peptide bond isoteres in the N-terminal region. Tourwe, D. (1985) Janssen. Chim. Acta 3,3; Hocart, S.J., et al. (1990) J. Med.Chem. 33, 1954-58.

This invention provides GRF analogs of increased potency possessing other features which provide technical advantages over previously reported GRF analogs. The compounds of the present invention are preferred for veterinary use, particularly in cattle, swine, sheep, poultry and fish.

For purposes of the present invention as disclosed and claimed herein, the following terms are defined as follows:
Ala -- the amino acid alanine.
Analog -- a compound which is structurally similar to another. When used in reference to polypeptides it refers to primary, secondary, or tertiary structure.
Arg -- the amino acid arginine.
Asn -- the amino acid asparagine.
Asp -- the amino acid aspartic acid.
Base pair (bp) -- refers to DNA or RNA. The abbreviations A,C,G, and T correspond to the 5'-monophosphate forms of the nucleotides (Deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA, heteroduplex base pair may refer to a partnership of T with U or C with G.
Cys -- the amino acid cysteine or one-half of a cystine residue covalently linked via a disulfide bridge to another one-half cystine residue.
DNA -- deoxyribonucleic acid.
EDTA -- an abbreviation for ethylenediamine tetraacetic acid.
ED₅₀ -- an abbreviation for median effective dose.
Functional analog -- refers to a molecule having similar functional properties but a modified structure relative to the naturally occurring form of that molecule or compound. A functional analog includes fragments of (or additions to) the parent molecule which has similar functional properties and reactivities as the parent molecule.
Gln -- the amino acid glutamine.
Glu -- the amino acid glutamic acid.
Gly -- the amino acid glycine.
GRF PEPTIDE -- a polypeptide comprising from 27 to 76 amino acid residues which promotes the release and synthesis of growth hormone from the pituitary gland. GRF PEPTIDEs include the natural or synthetic functional analogs thereof as disclosed in U.S. Patent Nos. 4,517,181, 4,518,586, 4,528,190, 4,529,595, 4,563,352, 4,585,756, 4,595,676, 4,605,643, 4,620,976, 4,626,523, 4,628,043, and 4,689,318; the entire teachings of which are incorporated by reference. The term GRF PEPTIDE includes the nontoxic salts thereof.
His -- the amino acid histidine.
Hse -- the amino acid homoserine.
Ile -- the amino acid isoleucine.
Leu -- the amino acid leucine
Lys -- the amino acid lysine.
Met -- the amino acid methionine or its deformylated analog.
mRNA -- messenger RNA.
MWCO -- an abbreviation for molecular weight cut-off.
Orn -- ornithine.
Phe -- the amino acid phenylalanine.
Plasmid -- a extrachromosomal self-replicating genetic element.
PMSF -- an abbreviation for phenylmethylsulfonyl fluoride.
Pro -- the amino acid proline.
Reading frame -- the nucleotide sequence from which translation occurs "read" in triplets by the translational apparatus of tRNA and ribosomes and associated factors each triplet corresponding to a particular amino acid. Because each triplet is distinct and of the same length, the coding sequence must be a multiple of three, a base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" being maintained.
Recombinant DNA Cloning Vector -- any autonomously replicating agent, including, but not limited to, plasmids and phages, comprising a DNA molecule to which one or more additional DNA segments can or have been added.
Recombinant DNA Expression Vector -- any recombinant DNA cloning vector in which a promoter has been incorporated.
Replicon -- a DNA sequence that controls and allows for autonomous replication of a plasmid or other vector.
RNA -- ribonucleic acid.
RP-HPLC -- an abbreviation for reverse-phase high performance liquid chromatography.
Ser -- the amino acid serine.
Thr -- the amino acid threonine.
Transcription -- the process whereby information contained in a nucleotide sequence of DNA is transferred to a complementary RNA sequence.
Translation -- the process whereby the genetic information of messenger RNA is used to specify and direct the synthesis of a polypeptide chain.
Tris -- an abbreviation for tris(hydroxymethyl)aminomethane.
Trp -- the amino acid tryptophan.
Tyr -- the amino acid tyrosine.
Val -- the amino acid valine.
Vector -- a replicon used for the transformation of cells in gene manipulation bearing polynucleotide sequences corresponding to appropriate protein molecules which when combined with appropriate control sequences confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. The term "vector" as used herein includes Recombinant DNA cloning vectors and Recombinant DNA expression vectors.

The restriction site and function maps presented in the accompanying drawings are approximate representations of the recombinant DNA vectors described herein. The restriction site information is not exhaustive; therefore there may be more restriction sites of a given type on the vector than are illustrated in the drawings.

Figure 1. A restriction site and function map of plasmid pHS190.

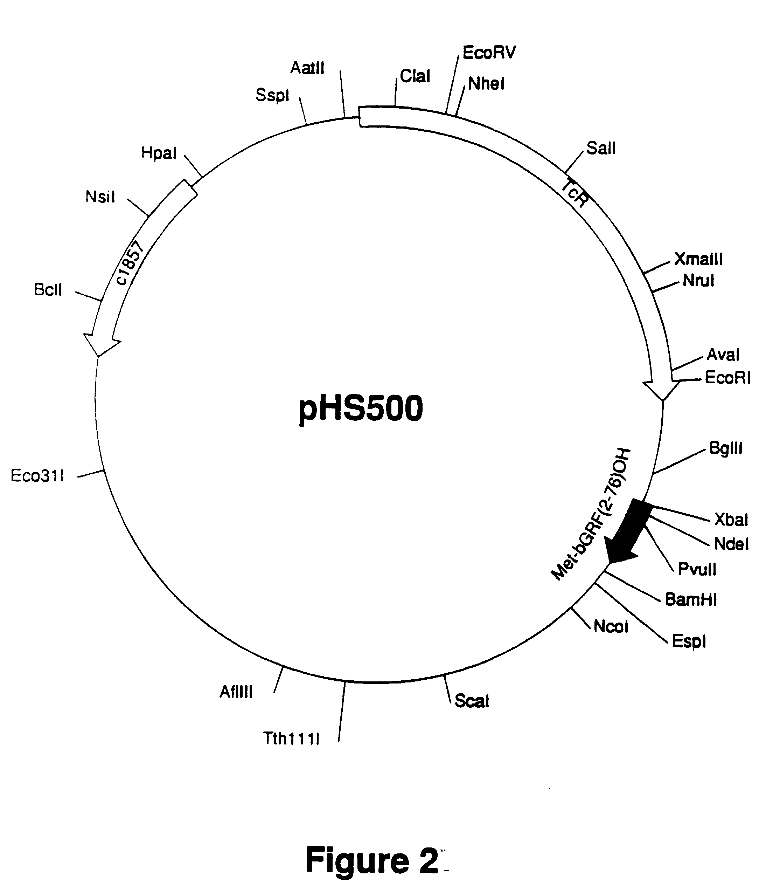

Figure 2. A restriction site and function map of plasmid pHS500.

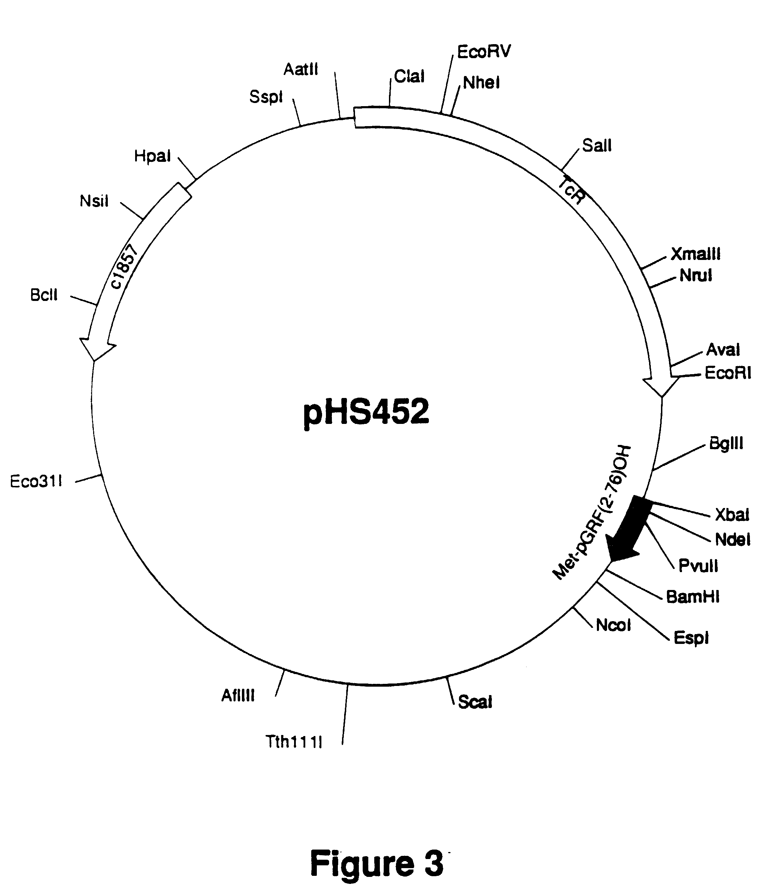

Figure 3. A restriction site and function map of plasmid pHS452.

Figure 4. A graphical representation indicating the growth hormone level elevation achieved in barrows via the administration of p-methyl hippuroyl pGRF(2-76)OH.

Figure 5. A graphical representation indicating the effects on urinary urea nitrogen level excretion in barrows in response to the administration of p-methyl hippuroyl pGRF(2-76)OH.

This invention provides synthetic GRF peptide analogs (GRF PEPTIDES) represented by the following formula 1: 
X1-Ala-Asp-Ala-Ile-Phe-Thr-Asn-A9-Tyr-Arg-A12
  Val-Leu-A15-Gln-Leu-Ser-Ala-Arg-A21-A22-Leu
  Gln-A25-Ile-A27-A28-Arg-Y-Z-T
 wherein
A9 = Ser, Ala, Leu, Thr or Asn,
A12 = Arg or Lys,
A15 = Gly, Ala, Thr or Leu,
A21 = Arg or Lys,
A22 = Ala or Leu,
A25 = Asp or Glu,
A27 = Met, Ser, Arg, Leu, or Norleucine,
A28 = Ser or Asn,

Y= 0, or an amino acid sequence from 1 to 15 amino acids;
Z = 0 or an amino acid sequence of from 1 to 32 amino acids;
   and
T= a carboxyl terminal group represented by the formula -COORₐ, -CRₐO, -CONHNHRₐ, -CON(Rₐ)(R_{b}) or CH₂ORₐ wherein Rₐ and R_{b} independently are lower alkyl, hydrogen, a Hse(lactone), HseOH or HseN(R_{c}) (R_{d}) wherein R_{c} and R_{d} independently are hydrogen or lower alkyl,
X1 is an acyl group represented by the formula 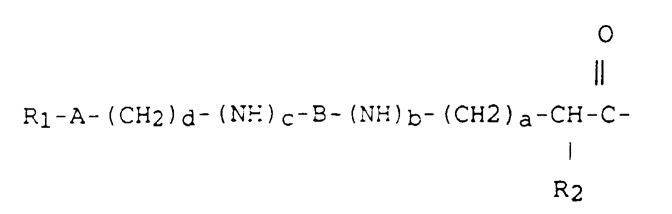
wherein
R₁ = p-methyl-phenyl,
a = zero,
b= 1,
c= zero,
d= zero,
A = zero,
B= carbonyl;
R₂ = H,
and the pharmaceutically acceptable non-toxic salts thereof.

In the definition of formula 1, the term "lower alkyl" refers to C₁-C₄ alkyl groups such as methyl, ethyl, n-propyl, isopropyl, n-butyl, sec-butyl, isobutyl and t-butyl; "lower alkoxy" refers groups such as methoxy, ethoxy, n-propoxy, t-butoxy and the like; "halo" refers to groups such as fluorine, chlorine, bromine and iodine; "substituted phenyl" refers groups such as phenyl substituted by one or two of the same or different groups selected from halo, lower alkyl, lower alkoxy, hydroxy, nitro, amino, acetamido or sulfonamido, exemplified by such groups as 4-chlorophenyl 3-iodophenyl, 2-fluorophenyl, 4-methylphenyl, 3 chloro-4-methylphenyl, 3- bromophenyl, 4-ethylphenyl, 3-ethoxyphenyl, 2-methoxyphenyl, 4-isopropoxyphenyl, 4-hydroxyphenyl, 3,4-dihydroxyphenyl, 2,4-dihydroxyphenyl, 4-nitrophenyl, 3-aminophenyl, 3-chloro-4-hydroxyphenyl, 2-acetamidophenyl, 4-sulfonamidophenyl, 3,4-dimethoxyphenyl, 2-fluoro-4-aminophenyl, and the like; "a 5 or 6 membered heterocycle containing one or more of N, O, or S" refers to the 5 membered heterocycles such as for example, pyrrole, thiophene, furan, imidazole, oxazole oxadiazole, thiazole, 1,3,4,-thiodiazole, asoxazole, and the like; and to 6-membered heterocycles such as for example, pyridine, pyrimidine, pyran, dihydropyran, thiazine, thiapyram, triazine and like 6-membered heterocycles. Such 5- and 6-membered heterocyles may also bear a substituent group such as lower alkyl, lower alkoxy, hydroxy, amino, or halo. Examples of acyl groups represented by X1 of formula 1 include p-chloro hippuroyl, p-methyl hippuroyl, p-nitro hippuroyl, hippuroyl, p-hydroxy hippuroyl, 3-benzoyl propionyl, n-phthaloyl glycyl, N-phenylmalonamidoyl, p-methyl--N-phenylmalonamidoyl, or p-fluorohippuroyl.

As previously mentioned hereinabove, preferred compounds of the invention are derivatives and modifications of animal GRF peptides.

The designation "A20", "A21", etc., in reference to amino acid residues of the compound of formula 1, corresponds to the number of the particular amino acid when numbered consecutively from the amino terminus of the protein as it occurs in nature and in the literature. Such uniform numbering of amino acid residues of well characterized proteins is well known by those of ordinary skill in the art. Designations such as "des-Tyr1-GRF" are widely understood in the art as meaning the natural GRF molecule lacking the amino-terminal tyrosine residue. Renumbering the remaining amino acid residues of the protein is unnecessary and may result in confusion. In the above example, it is understood that the amino acid occupying the second position in the naturally occurring protein would be the amino terminal residue but retain its designation as Ala2 for example. Designations according to the well accepted amino acid designations of the different GRF species will be followed herein.

A preferred peptide of the invention comprises a modification of porcine GRF (pGRF) which is represented by the formula 1 wherein: A9 = Asn, A12 = Arg, A15 = Thr, A21 = Arg, A22 = Leu, A25 = Asp, A27 = Leu, and A28 = Ser.

A further preferred peptide of the invention comprises a modification of bovine GRF (bGRF) which is represented by the formula 1 wherein A9 = Asn, A12 = Arg, A15 = Thr, A21 = Arg, A22 = Leu, A25 = Asp, A27 = Leu, and A28 = Asn.

A further preferred peptide of the invention comprises a modification of human GRF (hGRF) which is represented by the formula 1 wherein: A9 = Ser, A12 = Lys, A15 = Gly, A21 = Lys, A22 = Leu, A25 = Asp, A27 = Met, and A28 = Ser.

Preferred peptides of the invention comprise compounds of the formula 1 wherein Y is the 30-43 amino acid sequence of the mature GRF PEPTIDE. Preferred compounds of the invention comprise a compound of the formula 1 wherein Y is the 30-43 amino acid sequence of the naturally occurring 30-43 amino acid sequence of the particular species of GRF in question, i.e. pGRF, bGRF, hGRF, etc. Especially preferred Y peptides comprise the amino acid sequence: 
Gln-Gln-Gly-Glu-A34-A35-Gln-Glu-A38-A39-A40-Arg-A42-
Arg-Leu
 wherein:
A34 = Asp or Ser,
A38 = Arg or Gln,
A39 = Gly or Arg,
A40 = Ala or Ser,
A42 = Ala, Val, or Phe.

An especially preferred Y peptide comprises the amino acid sequence:

Glu Gln Gly Glu Ser Asn Gln Glu Arg
Gly Ala Arg Ala Arg Leu

when the particular form of GRF in question is bovine GRF.

An especially preferred Y peptide comprises the amino acid sequence: 
Gln Gln Gly Glu Arg Asn Gln Glu Gln
Gly Ala Arg Val Arg Leu
 when the particular form of GRF in question is porcine GRF.

An especially preferred Y peptide comprises the amino acid sequence: 
Glu Gln Gly Glu Ser Asn Gln Glu Arg Gly
Ala Arg Ala Arg Leu
 when the particular form of GRF in question is human GRF.

Preferred peptides of the invention are represented when z comprises the 44-76 amino acid sequence of GRF precursor PEPTIDES. Especially preferred is the 44-76 amino acid sequence of the naturally occurring 44-76 amino acid sequence of the GRF from the particular species under investigation.

A most preferred form of the peptide moiety represented by Z comprises the naturally occurring 44-76 amino acid GRF sequence of a particular species wherein the lysine residues thereof are replaced by arginine residues and the methionine residues are replaced by leucine residues. For example, an most preferred amino acid sequence of the GRF PEPTIDE exists when, Z comprises the amino acid sequence Gly-Arg-Gln-Val-Asp-Ser-Leu-Trp-Ala-Asp-Gln-Arg-Gln-Leu-Ala-Leu-Glu-Ser-Ile-Leu-Ala-Thr-Leu-Leu-Gln-Glu-His-Arg-Asn-Ser-Gln-Gly which is such a modification of porcine GRF and is preferred when the GRF species in question is porcine.

A most preferred amino acid sequence of the GRF PEPTIDE exists when, Z comprises the amino acid sequence: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly which is such a modification of bovine GRF and is preferred when the GRF species in question is bovine.

A most preferred Z sequence of the formula 1 is zero which is preferred when the GRF species in question is human.

Preferred compounds of the invention comprise compounds of the formula 1 wherein the Y and/or Z peptides are of a sufficient length such that high level expression in E. Coli is achieved.

Preferred compounds of the invention further comprise GRF PEPTIDES where none, one, or more than one of the amino acids having a free amino group is chemically modified. For purposes of the present invention, "chemically modified" is defined as the derivitization of an amino acid's free amino group with an alkyl or hydroxyalkyl group. The extent of modification is controlled by the length of reaction or the amount of reagent. It is preferred that all amino acids having a free amino group are chemically modified. Especially preferred is a GRF PEPTIDE which only has one free amino group that can be chemically modified. Such a compound has no lysine or methionine residues. Lysine and/or methionine-containing compounds are converted to compounds with a free amino group only at the N-terminus by replacing lysines with arginines and methionines with leucines.

The construction of the GRF compounds of this invention proceeds by first synthesizing the GRF PEPTIDE having a free amino group at the N-terminus and then acylating the N-terminus with the desired X1 acid or an active derivative thereof. GRF PEPTIDES may be made by solid phase peptide synthesis or by recombinant methods. Both methods are described in U.S. Patent 4,617,149, the entire teaching of which is herein incorporated by reference. Recombinant methods are preferred if a high yield is desired.

The principles of solid phase chemical synthesis of polypeptides are well known in the art and may be found in general texts in the area such as Dugas, H. and Penney, C., Bioorganic Chemistry (1981) Springer-Verlag, New York, pgs. 54-92. A description of the techniques and methodolgy of the solid phase synthesis of GRF analogs may be found in European Patent Application No. 85302975.9, Publication Number 0 161 852, published November 21, 1985, the entire teaching of which is hereby incorporated by reference.

In the preferred practice of the invention, synthesis of the GRF PEPTIDE is achieved by recombinant DNA technology. It is well known in the art how to construct a totally synthetic or semi-synthetic DNA sequence encoding a given amino acid sequence. Brown, et al., Methods in Enzymology 68:109 (1979). To effect the translation of the desired polypeptide, one inserts the engineered synthetic DNA sequence in any of a plethora of appropriate recombinant DNA expression vectors through the use of appropriate restriction endonucleases. The particular endonucleases employed will be dictated by the restriction endonuclease cleavage pattern of the parent expression vector to be employed. The GRF PEPTIDE coding sequence must be positioned so as to be in proper reading frame with the promoter and ribosome binding site of the expression vector, both of which are functional in the host cell in which the GRF PEPTIDE is to be expressed. It is further well known in the art the particular codons which may be used to achieve high level expression in a particular recombinant environment. See, W. Fiers, et al., (1976) Nature 260:500, and United States Patent No. 4,356,270.

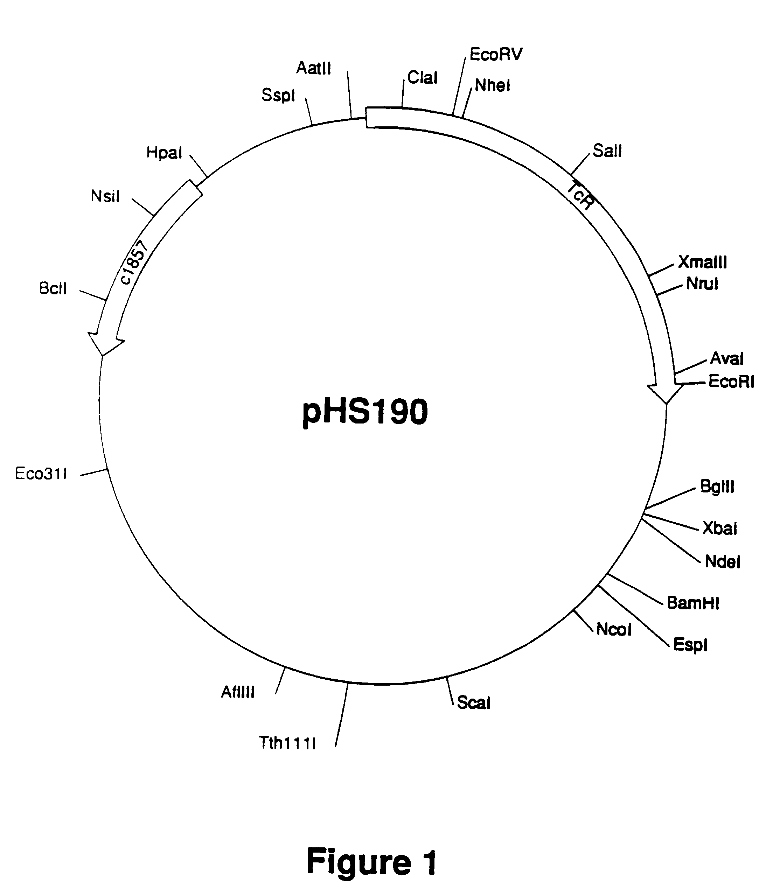

A preferred vector for expression in an E. coli host cell is derived from E. coli plasmid pHS190, which comprises the TcR gene, the lambda cI857 repressor and the lambda pL promoter. A restriction site and function map of pHS190 appears in Figure 1 of the accompanying drawings. Plasmid pHS190 is on deposit with the Northern Regional Research Laboratories under the accession number NRRL B-18410 (date of deposit: September 9, 1988).

The synthetic gene sequences of the present invention encode porcine, bovine, and human GRF analogs of the structure Met₁-Ala₂-pGRF(3-76)OH, Met₁-Ala₂-bGRF(3-76)OH, and Met₁-Ala₂-hGRF(3-76)OH. GRF analogs useful in a variety of other species such as horse, duck, rat, guinea pig, chinchilla, chicken, and others can be obtained with synthetic genes encoding the corresponding GRF PEPTIDE from the known amino acid sequences of these molecules and modifying them in a manner analogous to the bovine and porcine GRF PEPTIDES exemplified herein.

The action of methionyl amino peptidase (MAP), a protein indigenous to E. coli, will remove the N-terminal methionine of the above compounds intrinsically, producing the desired pGRF(2-76)OH, bGRF(2-76)OH, and hGRF(2-76)OH peptides. These (2-76)OH GRF PEPTIDES are then converted into the compounds of the invention (formula 1) by the acylation of the amino terminal amino group with an X1 acid or an activated derivative thereof. However, it is common in the art of molecular biology to express peptides as fusion proteins. In such cases where the GRF PEPTIDE is to be produced as a fusion protein and the GRF PEPTIDE later cleaved from the construct, one must engineer the coding sequence in a manner such that a protease (e.g., carboxypeptidase) or chemical (e.g. cyanogen bromide) cleavage site occurs between the GRF PEPTIDE and the heterologous peptide in the resulting fusion construct. The techniques for producing fusion proteins methods for achieve cleavage of exogenous heterologous peptide from fusion protein constructs are well known in the art in the art. See, e.g. United States Patent Nos. 4,366,246 and 4,425,437. In determining an appropriate proteolytic enzyme, it is preferred that the amino acid backbone of the fusion protein should be designed not to have any additional cleavage sites in the desired polypeptide (i.e. the GRF PEPTIDE) except at the interface between the desired peptide and the exogenous peptide. This avoids potential degradation of the desired GRF PEPTIDE. The amino acid sequence specificity of a wide variety of chemical and enzymatic agents is well known in the art. See, e.g.. Proteolytic Enzymes: A Practical Approach, Benyon, R.J. and Bond, J.S., eds (1989) Oxford University Press, Oxford, England, UK.

In the preferred practice of the invention as exemplified herein, the desired synthetic DNA sequence encoding the GRF PEPTIDE of interest is constructed so as to possess an XbaI site at the 5' end of the coding strand and a BamHI site at the 3' end of the coding strand. To generate the vector DNA, the EcoRI site of pHS190 is eliminated. The pHS190 vector is digested with EcoRI, the protruding ends are filled, and the plasmid religated. Subsequent digestion with BamHI and XbaI yields the vector DNA. The synthetic GRF PEPTIDE coding sequence is then inserted into the vector DNA and the vector religated.

Transformation of the ligated expression vector into a bacterial (e.g. E. coli) or mammalian host cell appropriate for the particular expression vector employed results in a recombinant cell capable of expressing a GRF PEPTIDE suitable for use in the practice of the instant invention. Suitable expression vectors for bacterial and mammalian expression environments may be found in well known laboratory manuals in the art such as Current Protocols in Molecular Biology, (1989 and supplements) Ausubel, et al., eds.,John Wiley and Sons, NY.

In the preferred embodiment of the invention exemplified herein, plasmid pHS 452 is prepared as described above by inserting the coding sequence for the Met₁-Ala₂-pGRF(3-76)OH GRF PEPTIDE. The DNA sequence encoding the Met₁-Ala₂-pGRF(3-76)OH GRF PEPTIDE and the corresponding amino acid sequence as provided herein is:

ATG GCT GAT GCT ATT TTT ACT AAT
Met Ala Asp Ala Ile Phe Thr Asn

AAT TAT CGA CGC GTT CTG ACT CAG CTG TCT
Asn Tyr Arg Arg Val Leu Thr Gln Leu Ser

GCT CGT CGT CTG CTG CAG GAT ATT CTG TCT
Ala Arg Arg Leu Leu Gln Asp Ile Leu Ser

CGT CAG CAG GGT GAA CGT AAC CAG GAA CAA
Arg Gln Gln Gly Glu Arg Asn Gln Glu Gln

GGA GCC CGT GTT CGT CTT GGT CGT CAG GTT
Gly Ala Arg Val Arg Leu Gly Arg Gln Val

GAT TCT CTG TGG GCT GAT CAA CGT CAG CTT
Asp Ser Leu Trp Ala Asp Gln Arg Gln Leu

GCT CTC GAG TCT ATC CTG GCT ACT CTG CTG
Ala Leu Glu Ser Ile Leu Ala Thr Leu Leu

CAG GAA CAT CGT AAT TCT CAG GGT TAA TAG
Gln Glu His Arg Asn Ser Gln Gly StopStop

When the DNA sequence which is inserted encodes the Met₁-Ala₂-bGRF(3-76)OH GRF PEPTIDE, the resulting plasmid is designated pHS500. The DNA sequence encoding, and the amino acid sequence corresponding to, the Met₁-Ala₂-bGRF(3-76)OH GRF PEPTIDE is:

ATG GCT GAT GCT ATT TTT ACT AAT TCT TAT
Met Ala Asp Ala Ile Phe Thr Asn Ser Tyr

CGT CGT GTC CTT ACT CAG CTG TCT GCG CGC
Arg Arg Val Leu Thr Gln Leu Ser Ala Arg

CGT CTG CTG CAG GAT ATC CTG AAT CGT CAG
Arg Leu Leu Gln Asp Ile Leu Asn Arg Gln

CAA GGT GAA CGT AAT CAG GAA CAA GGT GCT
Gln Gly Glu Arg Asn Gln Glu Gln Gly Ala

CGT GTA CGT CTG GGT CGC CAA GTT GAT GGT
Arg Val Arg Leu Gly Arg Gln Val Asp Gly

GGT TGG ACT GAT CAA CAG CAG CTT GCT CTC
Val Trp Thr Asp Gln Gln Gln Leu Ala Leu

GAG TCT ACA CTA GTT TCT CTG CTG CAG GAA
Glu Ser Thr Leu Val Ser Leu Leu Gln Glu

CTG CGG AAT TCT CAA GGT TAA TAG
Arg Arg Asn Ser Gln Gly StopStop

A DNA sequence may be inserted which encodes the Met₁-Ala₂-hGRF(3-44)OH GRF PEPTIDE. The DNA sequence encoding, and the amino acid sequence corresponding to, the Met₁-Ala₂-hGRF (3-44)OH GRF PEPTIDE is: 
ATG GCT GAC GCT ATC TTC ACT AAC TCT TAC CGT
Met Ala Asp Ala Ile Phe Thr Asn Ser Tyr Arg

AAA GTT CTG GGT CAG CTG TCT GCT CGT AAA CTG
Lys Val Leu Gly Gln Leu Ser Ala Arg Lys Leu

CAG GAC ATC ATG TCT CGT GAG CAG GGT GAA TCT
Gln Asp Ile Met Ser Arg Glu Gln Gly Glu Ser

AAC CAG GAA CGT GGT GCT CGT GCT CGT CTG TAA
Asn Gln Glu Arg Gly Ala Arg Ala Arg Leu Stop
 Cravador, A, et al. (1985) Biochimie 67:829-834.

In the preferred practice of the invention as exemplified herein where the expression vector uses the pHS190 backbone, the host cell is grown at 32°C until it is desired to express the polypeptide compound. The cI857 repressor becomes inactivated upon a shift in growth temperature to approximately 42°C and the pL promoter is derepressed. A large amount of polypeptide having GRF activity is then produced at levels up to about 15% of total cell protein. The active polypeptide can be purified by techniques well-known to skilled artisans and as set out in the following Examples. As stated previously, the indigenous action of MAP will remove the N-terminal methionine residue yielding the GRF(2-76)OH GRF Peptide. However, following purification of the protein, any remaining N-terminal methionine residues may be removed by conventional enzymatic methods such as through the action of an appropriate amino-peptidase, or other chemical means such as through the action of cyanogen bromide.

The claimed compounds of the present invention are prepared by the N-acylation of the amino terminus of the (2-76)OH GRF PEPTIDES with an active ester of the X1 carboxylic acid desired. Acylation of the amino terminal nitrogen of the (2-76)OH GRF PEPTIDE is preferably carried out via succinimidyl esters of the X1 acid. A nucleophilic attack by the amino terminal nitrogen of the (2-76)OH GRF PEPTIDE on the carbonyl carbon of the ester linkage of the succinimidyl ester results in acylation. The following reaction diagram illustrates the acylation resulting in the synthesis of hippuroyl-GRF(2-76)OH:

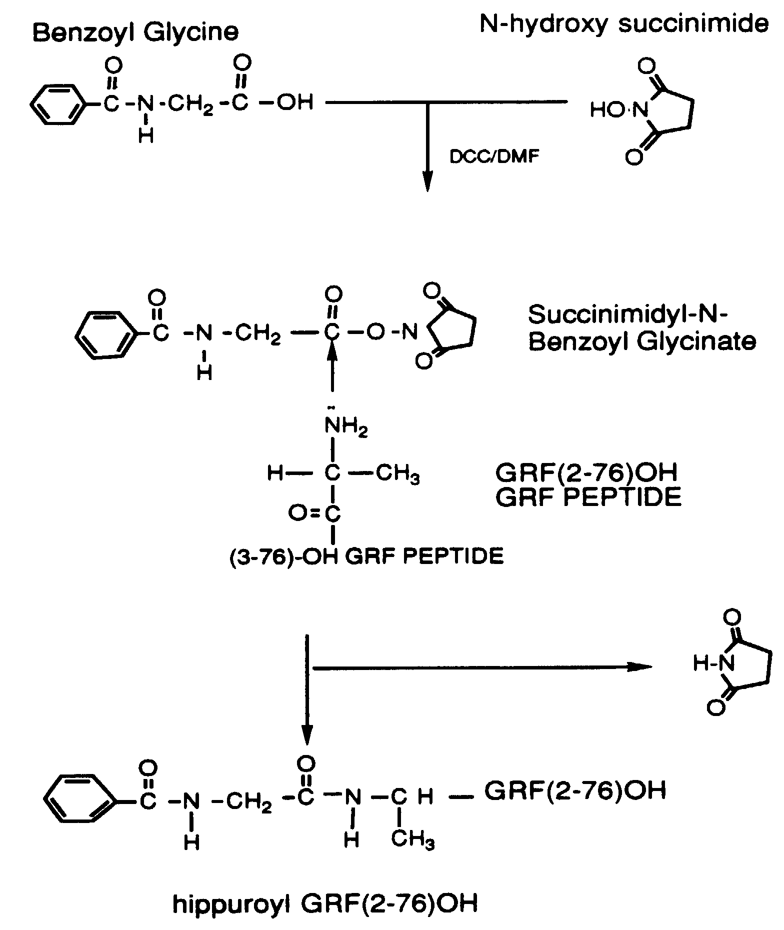

Other active esters of the X1 carboxylic acid can be used in the acylation. For example, active esters formed with the chloroformates such as ethyl chloroformate and isobutyl chloroformate or with N-hydroxyheterocycles such as HoBT(hydroxybenzotriazole) can be used. Other active derivatives conventionally used for the acylation of amino acids or for the coupling of amino acids can be used. These could include symmetric or N-carboxy anhydrides.

The acylation is carried out in an aqueous medium at a pH between about 7.5 and 9.5 and at a temperature between about 15 and 35°C. Alternatively, an aprotic solvent such as DMSO may be used.

Examples of X1 acids used to prepare the GRF compounds of the formula 1 include hippuric acid, p-methylhippuric acid, p-chlorohippuric acid, p-nitrohippuric acid, p-hydroxyhippuric acid, phenyl amino carbonic acid, 4-hydroxyphenyl pyruvic acid, p-fluoro hippuric acid, N-phenylmalonic acid amide, p-methyl-N-phenyl malonic acid amide, N-(2-thienoyl)glycine, N-(2-furoyl)glycine, N-(2-furoyl)-b-aminopropionic acid, N-(3-thienoyl)-a-aminopropionic acid, N-(3-pyridinoyl) glycine, N-4-pyridinyl) malonamide acid, N-(1,3 thiazinyl) malonamide acid, N-(1,3 thiazinyl) succinamide, N-phenyl glutaramide acid, N-[2-methoxyethyl)aminosulfonyl] glycine and sulfinyl, N-[(2-ethylthioethyl)aminosulfonyl] glycine and sulfinyl, and N-(2-hydroxyethyl)malonamide acid when acylating a (2-29,44, or 76)GRF PEPTIDE.

It will be readily apparent to those of ordinary skill in the art that the compounds of the present invention may also be prepared beginning with a (3-29,44, or 76) GRF PEPTIDE, a GRF(4-29,44, or 76) GRF PEPTIDE, etc., free or attached to a support with a similar N-terminally blocked peptide or substituted carboxylic acid derivative.

Included in the compounds of this invention are their pharmaceutically acceptable non-toxic salts of the acylated GRF PEPTIDES. The term "pharmaceutically acceptable non-toxic salts" encompasses both organic and inorganic acid addition salts including, for example, those prepared from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. Preferably, the acid addition salts are those prepared from hydrochloric acid, acetic acid, or succinic acid. Any of the above salts is prepared by conventional methods. The term also includes "carboxylic acid salts", for example, amine, ammonium, quaternary ammonium, alkali metal and alkaline earth metal salts such as calcium, magnesium, sodium, potassium, and lithium, and the like, formed with any carboxy group present.

The following Tables 1, 2 and 3 illustrate the enhanced activity of compounds of the instant invention:

**Table 1**

| Enhanced Growth Hormone-Releasing Activity of bGRF (1-77) OH by substitutions for Tyr-1* | |
|---|---|
| Peptide | EC50 (nM)** |
| | |
| bGRF (1-77)0H | 0.59 |
| Hippuroyl-1 bGRF(2-77)0H | 0.86 |
| p-Hydroxy Hippuroyl bGRF(2-77)0H | 0.33 |
| p-Chloro Hippuroyl bGRF(2-77)OH | 0.19 |
| p-Fluoro Hippuroyl bGRF(2-77)0H | 0.14 |
| p-Nitro Hippuroyl bGRF (2-77)0H | 0.51 |
| p-Methyl Hippuroyl bGRF(2-77)OH | 0.05 |
| n-Phenylmalonamindoyl bGRF(2-77)0H | 0.19 |

**Table 2**

| Enhanced Growth Hormone-Releasing Activity of bGRF (1-77)OH by substitutions for Tyr-1* | |
|---|---|
| Peptide | EC50 (nM)** |
| des Tyr-1 pGRF(2-76)0H | 95.0 |
| Hippuroyl-1 pGRF(2-76)OH | 0.28 |
| p-Methyl Hippuroyl pGRF(2-76)OH | 0.06 |
| p-Methyl Phenylmalonamindoyl pGRF(2-76)OH | 0.06 |

**Table 3**

| In Vitro Potency of p-Methyl Hippuroyl-1 pGRF Analogs of Different Lenght* | |
|---|---|
| Peptide | EC50 (nM)** |
| p-Methyl Hippuroyl pGRF(2-29)NH2 | 0.02 |
| p-Methyl Hippuroyl pGRF(2-44)OH | 0.07 |
| p-Methyl Hippuroyl pGRF(2-76)OH | 0.06 |

| | |
|---|---|
| * Growth Hormone Released into media from rat primary pituitary cells was measured after exposure to 7 doses of secretagogue for 3h. | |
| ** EC50 is the calculated median effective dose. | |

The present invention also provides a pharmaceutical composition comprising as the active agent a polypeptide compound represented by the formula 1 or a pharmaceutically acceptable non-toxic salt thereof and a pharmaceutically acceptable solid or liquid carrier.

In administering the polypeptide compounds of this invention parenterally, the pharmaceutical forms suitable for injection include sterile aqueous solutions or dispersions and sterile powders for reconstitution into sterile injectable solutions or dispersions. The carrier can be a solvent or dispersing medium containing, for example, water, ethanol, polyol (for example glycerol, propylene glycol, liquid polyethylene glycol, and the like), suitable mixtures thereof, and vegetable oils. Proper fluidity can be maintained, for example, by the use of a coating such as lecithin, by the maintenance of the required particle size in the case of dispersion and by the use of surfactants. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin.

Sterile injectable solutions can be prepared by incorporating the compounds of this invention in the required amount of the appropriate solvent with various of the other ingredients, as desired. If desired, and for more effective distribution, the compounds can be incorporated into slow release or targeted delivery systems such as polymer matrices, liposomes, and microspheres. The compounds may be delivered via mechanical release devices, osmotic pumps, or any other release device or system which provides continuous or pulsatile delivery.

For intravenous (IV) use, a water soluble form the compound can be dissolved in one of the commonly used intravenous fluids and administered by infusion. Such fluids as, for example, physiological saline, Ringer's solution or 5% dextrose solution can be used. A suitable insoluble form of the compound may be prepared and administered as an aqueous base or a pharmaceutically acceptable oil base, e.g. an ester of a long chain fatty acid such as ethyl oleate.

The unit dosage form of the compound can be a solution of the compound, preferably in its salt form, in a suitable diluent in sterile, hermetically sealed ampoules. The concentration of the compound may vary, e.g. from about 1 percent to about 50 percent depending on the particular form of the compound, its solubility and the dosage desired in the application.

Also provided in the present invention is a method for inducing growth hormone release which comprises administering an effective amount of a polypeptide compound represented by the formula 1 or a pharmaceutically acceptable non-toxic salt thereof and a pharmaceutically acceptable solid or liquid carrier.

Doses of the compounds of this invention are administered to the recipient for a period during which stimulation of the release of growth hormone is desired. The weight of the recipient and mode of administration will have an influence upon the size of the dose necessary to induce a particular response. Preferred doses in sheep are about 0.01-3.0 mg/kg/day; a most preferred dose is about 0.5 mg/kg/day. Preferred doses in swine are about 3 µg/kg/day to 10 µg/kg/day. A most preferred dose in swine is about 3 µg/kg/day. For cattle preferred doses are about 0.5-12 mg/kg/day. An especially preferred dose is 3 mg/kg/day. Preferred doses in humans are about 0.03 mg/kg/day to 1.0 mg/kg/day. A most preferred dose is about 0.3 mg/kg/day.

It is especially advantageous to formulate the compounds of this invention in unit dosage form for ease of administration and uniformity of dosage. Unit dosage form as used herein refers to physically discrete units suited as unitary dosages for the subject to be treated. Each unit contains a predetermined quantity of the compound calculated to produce the desired therapeutic effect in association with the pharmaceutically acceptable carrier. The specific unit dosage form is dictated by and directly dependent upon (a) the unique characteristics of the particular composition and (b) the particular therapeutic effect to be achieved.

By definition, GRF and preferably the GRF compounds of the instant invention, when appropriately administered in a pharmaceutically acceptable form, result in an increase in the production of growth hormone in mammmals. Consequently, medical and agricultural applications of mammalian growth hormones may be similarly achieved by administration of compounds of the instant invention. Examples of disease states currently indicated for treatment by administration of growth hormone include dwarfism and osteoporosis. Other beneficial effects of growth hormone, such as increasing lean muscle mass, fostering wound-healing, and countering the effects of aging, may also be achieved by administration of the compounds of the instant invention.

The polypeptide compounds of the invention may be assayed in numerous systems. One in vivo assay is carried out in sodium pentobarbital-anesthetized rats. Wehrenberg et al., Biochem. Biophys. Res. Commun. 109:382 (1982). Growth hormone release is measured in vitro with rat anterior pituitary cells. Heiman et al., Endocrinol. 116:410 (1985).

The following examples are provided to further illustrate the invention described herein but are not intended to be limitations thereof.

### EXAMPLE 1.

### SOLID PHASE PREPARATION OF bGRF (2-77) OH.

GRF peptides were synthesized by solid-phase methodology utilizing an Applied Biosystems 430A peptide synthesizer (commercially available from Applied Biosystems, Foster City California) and synthesis cycles supplied by Applied Biosystems. Boc amino acids and other reagents were supplied bY Applied Biosystems and other commercial sources. Sequential Boc chemistry using double couple protocols are applied to the starting p-methyl benzhydryl amine resins for the production of C-terminal carboxamides. For the production of C-terminal acids , the corresponding PAM resin is used. Asparagine, Glutamine, Arginine, [α-(p-hydroxyphenyl) acetic acids], [β-(p-hydroxyphenyl) propionic acids], p-hydroxybenzoic acids, p-hydroxycinnamic acids, p-hydroxyphenoxy acetic acids are coupled using preformed hydroxy benzotriazole esters. All N-terminal additions were made using HOBT activated amino acids in the solid phase synthesis.

The following side chain protection is used:
Arg, Tosyl
Asp, cyclohexyl
Glu, cyclohexyl
Ser, Benzyl
Thr, Benzyl
Tyr, 4-bromo carbobenzoxy

Boc deprotection is accomplished with trifluoroacetic acid (TFA).in methylene chloride. Following completion of the synthesis the peptides are deprotected and cleaved from the resin with anhydrous hydrogen fluoride containing 10% meta-cresol. Cleavage of the side chain protecting group(s) and of the peptide from the resin is carried out at zero degrees centigrade or below, preferably -20°C for thirty minutes followed by thirty minutes at 0°C. After removal of the HF, the peptide/resin is washed with ether, and the peptide extracted with glacial acetic acid and lyophilized. Purification is accomplished by size-exclusion chromotography on a Sephadex G-10 (Pharmacia) column in 10% HOAc.

### EXAMPLE 2. RECOMBINANT PRODUCTION OF GRF PEPTIDES

### 2.A. RECOMBINANT EXPRESSION OF GRF (2-76) OH

### 2.A.1. Synthesis of Plasmid pHS452 Encoding Met₁pGRF (2-76) OH

Plasmid pHS452 is a recombinant DNA expression vector which produces large amounts of the preferred pGRF(2-76)OH polypeptide compound of the invention when the host cell is cultured under the proper conditions.

A lyophil of E. coli K12 RV308/pHS190 can be obtained from the Northern Regional Research Laboratories (NRRL), Peoria, IL 61604, under the accession number NRRL B-18410 (date of deposit: September 9, 1988) and used directly as the "culture" in the process below. A restriction site and function map of plasmid pHS190 is presented in Figure 1 of the accompanying drawings. Ten milliliters of TY broth (10 g tryptone, 5 g NaCl, and 5 g yeast extract per liter) containing 5 mg/ml of tetracycline is inoculated with a culture of E. coli K12 RV308/pHS190 and incubated with aeration at 30°C overnight (15-18 hours). The resulting culture is used as a source of plasmid pHS190.

One liter of TY broth containing 5mg/ml tetracycline is inoculated with a culture of E. coli K12 RV308/pHS190 and incubated with aeration at 32°C overnight (15-18 hours). The culture is then centrifuged at 5200 rpm in a Sorvall (DuPont Co., Instrument Products, Biomedical Division, Newtown, CT 06470) GSA rotor for 10 minutes at 4°C to pellet the cells. The resulting supernatant is discarded. The cell pellet is resuspended in 28 ml of a solution of 25% sucrose and 50 mM EDTA (ethylenediamine tetraacetic acid). About 1 ml of a solution of 20 mg/ml lysozyme in 0.25 M Tris-HCl (tris(hydroxymethyl)aminomethane hydrochloride), pH = 8.0, and about 1.5 ml of 0.5 M EDTA, PH = 8.0, are added to and mixed with the cell suspension. The resulting mixture is incubated on ice for 15 minutes. Three milliliters of lysing solution (prepared by mixing 3 ml of 10% Triton® X-100 (Rohm & Haas); 75 ml of 0.25 M EDTA, pH = 8.0; and 7 ml of water) are added to the lysozyme-treated cells with gentle mixing. The resulting solution is incubated on ice for another 15 minutes.

The cellular debris is removed from the solution by centrifugation at 17,000 rpm in a Sorvall SS34 rotor for about 45 minutes at 4°C. About 28.6 g of CsCl and ∼1 ml of a 5 mg/ml ethidium bromide solution are added to the ∼30 ml of supernatant. Then, the volume is adjusted to 40 ml with water and the solution decanted into an ultracentrifuge tube. The tube is sealed, and the solution is centrifuged at 49,500 rpm in a Ti70 rotor (Beckman, 7360 N. Lincoln Avenue, Lincolnwood, IL 60646) for ∼18 hours. The resulting plasmid band, visualized with ultraviolet light, is isolated, extracted with CsCl-saturated isopropanol to remove the ethidium bromide, and dialysed against three changes of ∼20 volumes of TE buffer (10 mM Tris-HCl, pH = 7.5, and 1 mM EDTA). The dialysate is collected; then, two volumes of ethanol and 0.05 volumes of 3 M sodium acetate solution are added. The ethanol mixture is cooled to -20°C, and the plasmid DNA is pelleted by centrifugation at 10,000 rpm for 30 minutes in an SS34 rotor at -10°C. The resulting pellet is resuspended in ∼1 ml of TE buffer and then extracted with an equal volume of a phenol:chloroform mixture (1:1, v/v). The DNA in the aqueous phase is recovered by the addition of 0.1 volume of 3 M sodium acetate and 2 volumes of ethanol, followed by incubation at -20°C for ∼30 minutes and centrifugation at 15,000 rpm in an SS34 rotor for 20 minutes. The resulting DNA pellet is rinsed first with 70% ethanol and then with 100% ethanol and dried.

The ∼1.0 mg of plasmid pHS190 DNA obtained by the above procedure is suspended in 1.5 ml of 0.1X TE buffer and stored at -20°C. About 10mg of plasmid pHS190 DNA are digested with restriction enzyme EcoRI (∼10 units) in a reaction containing the DNA in EcoRI buffer (100 mM Tris-HCl, pH = 7.5, 5 mM MgCl, 50 mM NaCl). The reaction is incubated for 2 hours at 37°C.

To convert the 5' overhanging ends to blunt ends 0.5 mM of each dNTP (dATP, dCTP, dGTP, and TTP) is added along with 1-5 units Klenow fragment (Boehringer Mannheim Biochemicals, 7941 Castleway Dr., P.O. Box 50816, Indianapolis, IN 46250). The reaction is incubated at 30°C for 15 minutes, then the enzyme is inactivated by treatment at 75°C for 10 minutes. The reaction mixture is extracted with phenol, phenol/chloroform, chloroform and then ethanol precipitated.

The plasmid is then resuspended in 50 ml of a solution containing 40 mM Tris HCl, PH 7.5, 10 mM MgCl, 10 mM dithiothreitol (DTT), 0.5 mM adenosine triphosphate, and 1 U T4 DNA ligase (Boehringer-Mannheim Biochemicals, 7941 Castleway Drive, Indianapolis, IN 46250). The reaction is incubated at 14°C overnight.

The ligated mixture was transformed into E. coli K12 RV308 (available from the NRRL as NRRL B-15624) by the following procedure from Maniatis et al., Molecular Cloning, pg. 250-251 (Cold Spring Harbor Press, 1982). One hundred milliliters of TY broth in a 500-ml flask was inoculated with l ml of an overnight culture of RV308. The cells were grown with vigorous shaking at 37°C to a density of ∼5 x 10 cells/ml. The culture was placed on ice for ten minutes, then centrifuged at 4000 x g for 5 minutes at 4°C. The cells were resuspended in 50 ml ice-cold 50 mM CaCl in 10 mM Tris-HCl, pH = 8.0. The cells were again incubated on ice for 15 minutes and recentrifuged. The cells were then resuspended in 3.3 ml of the calcium chloride solution. The ligation mixture was added to 200 ml of cells and incubated on ice for 30 minutes. The cells were then transferred to a 42°C water bath for 2 minutes. One ml of TY broth was added to the tube and the cells were incubated for one hour at 30°C. Two hundred microliter aliquots were then plated onto TY agar (TY broth + 1.5% agar) plates containing 5 mg/ml tetracycline and grown overnight at 30°C.

The plasmid is then resuspended in 10 ml of water. The vector is generated by digesting the plasmid with XbaI and BamHi. About 1 ml of XbaI (∼10 units) is added to 10 ml plasmid DNA (∼10 mg) and 5 ml 1OX XbaI buffer (500 mMTris-HCl, pH = 8.0, 100 mM MgCl, and 500 mM NaCl). After incubation at 37°C for 90 minutes, 0.5 ml of 5 M NaCl and 1 ml BamHI (10 units) are added and the incubation continued at 37°C for an additional 90 minutes. The reaction mixture is then subjected to agarose gel electrophoresis, and the ∼5.75 kb XbaI-BamHi vector fragment is isolated by electroelution followed by ethanol precipitation.

The following DNA sequence was synthesized on an Applied Biosystems Model 380B synthesizer using techniques well-known to one skilled in the art. The process by which construction of the synthetic gene is achieved by division of the sequence into oligonucleotides and subsequent ligation of these oligonucleotids was achieved in substantial accordance with the procedure of Brown, et al., Methods in Enzymology 68:109 (1979). The following DNA sequence encodes a preferred polypeptide of the invention wherein: A9 = Asn, A12 = Arg, A15 = Thr, A21 = Arg, A22 Leu, A25 = Asp, A27 = Leu, A28 = Ser, A38 = Gln, A39 = Gly, A40 = Ala, A42 = Val, and Z = Gly-Arg-Gln-Val-Asp-Ser-Leu-Trp-Ala-Asp-Gln-Arg-Gln-Leu-Ala-Leu-Glu-Ser-Ile-Leu-Ala-Thr-Leu-Leu-Gln-Glu-HiS-Arg-Asn-Ser-Gln-Gly, the positive strand of said sequence comprising:

ATG GCT GAT GCT ATT TTT ACT AAT AAT TAT CGA CGC GTT

CTT ACT CAG CTG TCT GCT CGT CGT CTG CTG CAG GAT ATT

CTG TCT CGT CAG CAG GGT GAA CGT AAC CAG GAA CAA GGA

GCT CGT GTT CGT CTT GGT CGT CAG GTT GAT TCT CTG TGG

GCT GAT CAA CGT CAG CTT GCT CTC GAG TCT ATC CTG GCT

ACT CTG CTG CAG GAA CAT CGT AAT TCT CAG GGT TAA TAG

The DNA comprising the coding sequence was then mixed with and ligated to the XbaI-BamHI vector fragment constructed above. The ligated mixture was then transformed in E. coli K12 RV308 as described above. The plasmid DNA of tetracycline- resistant transformants was analyzed by restriction enzyme digestion to verify that the plasmid was pHS452.

### 2.A.2. Synthesis of Plasmid pHS500 Encoding Met₁-bGRF (2-76) OH

The bovine derived recombinant GRF PEPTIDES were produced in substantial accordance with the teaching of Example 2.A.1. above. However, producing the recombinant bovine GRF PEPTIDE, the DNA sequence encodes a preferred polypeptide of the invention wherein: A9 = Ser, A12 = Arg, A15 = Thr, A21 = Arg, A22 = Leu, A25 = Asp, A27 = Leu, A28 = Asn, A38 = Gln, A39 = Gly, A40 = Ala, A42 = Val, and Z = Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly, the positive strand sequence of this DNA sequence being:

ATG GCT GAT GCT ATT TTT ACT AAT TCT TAT CGT CGT GTC

CTT ACT CAG CTG TCT GCG CGC CGT CTG CTG CAG GAT ATC

CTG AAT CGT CAG CAA GGT GAA CGT AAT CAG GAA CAA GGT

GCT CGT GTA CGT CTG GGT CGC CAA GTT GAT GGT GTT TGG

ACT GAT CAA CAG CAG CTT GCT CTC GAG TCT ATA CTA GTT

TCT CTG CTG CAG GAA CGT CGG AAT TCT CAA GGT TAA TAG

The DNA comprising the coding sequence was then mixed with and ligated to the XbaI-BamHI vector fragment as described in the teaching of Example 2.A.1. above. The ligated mixture was then transformed in E. coli K12 RV308 as described above. The plasmid encoding the bGRF sequence was denoted pHS500. The plasmid DNA of tetracycline-resistant transformants was analyzed by restriction enzyme digestion to verify that the plasmid was pHS500.

### 2.A.3. Synthesis of DNA Encoding Met₁-hGRF (2-46) OH

The human derived recombinant GRF PEPTIDES are produced in substantial accordance with the teaching of Example 2.A.1. above. However, in the preferred practice of the invention, when producing a recombinant human GRF PEPTIDE, a modified hGRF peptide is used wherein: A9 = Ser, A12 = Lys, A15 = Gly, A21 = Lys, A22 = Leu, A25 = Asp, A27 = Met, and A28 = Ser, Y comprises the amino acid sequence: Glu-Gln-Gly-Glu-Ser-Asn-Gln-Glu-Arg-Gly-Ala-Arg-Ala-Arg-Leu, and Z = zero, the positive strand sequence of the DNA sequence encoding this peptide comprises the sequence:

ATG GCT GAC GCT ATC TTC ACT AAC TCT TAC CGT AAA
GTT CTG GGT CAG CTG TCT GCT CGT AAA CTG CTG CAG
GAC ATC ATG TCT CGT GAG CAG GGT GAA TCT AAC CAG
GAA CGT GGT GCT CGT GCT CGT CTG TAA

The DNA comprising the coding sequence is then mixed with and ligated to the XbaI-BamHI vector fragment as described in the teaching of Example 2.A.1. above. The ligated mixture is then transformed in E. coli K12 RV308 as described above.

### EXAMPLE 2.B. ISOLATION OF RECOMBINANT GRF PEPTIDES

### 2.B.1. Expression of the GRF Analog in E. coli

E. coli K12 RV308/pHS451 was grown in TY broth containing 5 mg/ml tetracycline at 32°C until the cells reached mid-log phase. The temperature of the mixture was raised to 42°C and incubation continued for about 3 more hours. The cI857 temperature-sensitive repressor of the lambda pL promoter, positioned to drive GRF analog expression on plasmid pHS451, is inactivated at 42°C, thus allowing the expression of the GRF analog. The bGRF PEPTIDE is expressed in substantial accordance with the method described above for pGRF except that the plasmid pHS500 is employed in place of pHS452.

### 2.B.2. Isolation of Granules Containing the GRF Analog

When using a high level E. coli expression system as exemplified herein, the protein product is sequestered in inclusion bodies or granules. The granules containing the GRF analog are isolated and solubilized to isolate the GRF peptide of interest. First, the whole cells are centrifuged, then resuspended in water at 10°C. The cell slurry is homogenized in a Gaulin homogenizer at 8000 psig. The homogenized slurry is then diluted in water and agitated for 10 minutes, followed by adjustment of the pH to 8.4-8.6 with 10% sodium hydroxide. The mixture is then centrifuged. The solids represent the GRF analog-containing granules, which are frozen at -70°C until further use.

### 2.B.3. Final Purification of the GRF Analog

The granules prepared in Example 2.B.2. above are thawed at 2-5°C. The granules are solubilized by the addition of ten volumes of 0.05 N acetic acid-7M urea followed by homogenization for 5-8 minutes. The pH is then adjusted to 2.5-2.6 by the addition of 10% hydrochloric acid. The mixture is agitated 12-15 hours at 2-5°C. The solution is clarified by filtration through a Sparkler filter coated with Dicalite Speedex filter aid (Grefco, Torrance, CA). The conductivity of the solution is reduced to less than 4000 mohms by dilution with the acetic acid-urea solution.

A cation exchange column is prepared using S Sepharose® (Pharmacia, 800 Centennial Ave., Piscataway, NJ 08854) resin. The column contains one liter of resin per 50 g of material. The material is added to the column at a flow rate of 0.1 1/cm/hr and washed with 2 column volumes of 0.1 M sodium chloride in the acetic acid-urea solution. The GRF analog is eluted by a linear gradient of 0.25 M to 1.6 M sodium chloride in acetic acid-urea using three column volumes of each with 0.1 column volume fractions collected. The GRF analog-containing fractions are identified by conductivity, O.D.₂₇₆, HPLC and polyacrylamide gel electrophoresis. The fractions are then pooled.

An equal volume of acetic acid-urea solution is added to the pooled fractions. The material is then applied to a column containing S Sepharose® resin in acetic acid-urea sized to accommodate 50 g of protein per liter of resin. The flow rate is 0.02 1/cm²/hr. The GRF analog fractions are eluted by a linear gradient of 0.25 M to 1.2 M sodium chloride in acetic acid-urea. Fractions of 0.1 column volume are collected. The fractions are analyzed as before and the GRF analog-containing fractions are pooled.

A Sephadex® G-15 (Pharmacia) column is prepared in 0.02 M glycine, pH 2.5 with a column volume five times the volume of the previously pooled fractions. Fractions containing the O.D.₂₇₆ peak are isolated.

A column containing SP20SS resin (Sephabeads®, Mitsubishi Chemical, Tokyo) in 10% acetonitrile-0.02 M glycine, pH 2.5, is then prepared. The pooled GRF analog-containing solution is made 10% in acetonitrile and added to the column at a flow rate of 1.5-2 column volumes per hour. The column is washed with 2 column volumes of the acetonitrile-glycine buffer. The GRF analog is eluted by a gradient formed by three column volumes of 10% acetonitrile-0.02 M glycine mixed with three column volumes 50% acetonitrile-0.02 M glycine. Fractions of 0.1 column volume are collected and assayed for the GRF analog.

The GRF analog-containing material is then chromatographed over a Sephadex® G15 column equilibrated in 0.25 M acetic acid. The O.D.₂₇₆ peak is then isolated and lyophilized until further use.

### EXAMPLE 3. PREPARATION OF HIPPUROYL-1-pGRF(2-76) OH.

3.58 g. of benzoylglycine (commercially available from Transworld Chemicals) was dissolved in approximately 20 ml of DMF with stirring and cooled in an ice bath. To the solution was added 2.5 g of N-hydroxysuccinimide followed by 4.5 g dicyclohexyl carbodiimide (DCC). The reaction was stirred overnight with warming to room temperature.

The insoluble dicyclohexyl urea (DCU) formed by the reaction was removed by filtration and the DMF filtrate concentrated in vacuo. The residue was diluted with CH₂Cl₂. A precipitate formed which was filtered and dried in vacuo to give 3.79 grams of succinimidyl hippuroate.

Approximately 72 mg of pGRF(2-76)OH prepared in substantial accordance with the teaching of Example 2.A. above, was dissolved in 3 ml of 0.1 M Tris-HCl, pH7.8, 30% propanol with stirring at room temperature. To the reaction 57 mg of succinimidyl N-benzoyl glycinate was added and the reaction stirred at room temperature while removing 5 µl samples at 15 min, 1 hour and 2 hours after introduction of the succinimidyl n-benzoyl glycinate to follow the progress of the reaction. Each of these samples was diluted to 500µl with 0.1% TFA (.15-.2 mg/ml) and injected onto a 0.46 x 15 cm Vydac C18 column for RP-HPLC analysis and comparison with the starting material.

After 2.5 hours the reaction mixture was acidified with approximately 1 ml of glacial HOAc and applied to a 2.2 x 28.5 cm Sephadex G-10 column. The column was eluted with 10% HOAc and 7.5 ml fractions were collected. The ABS₂₈₀ of each fraction was determined to indicate the presence of peptide. Fractions 7 through 10 indicating the presence of peptide were combined, frozen, and lyophilized to give 70 mg of lyophilisate.

Approximately 9 mg of the sample was dissolved in 1.0 ml of 0.1 % TFA. The solution was divided into ten 0.1 ml aliquots which were frozen and lyophilized. A sample of the lyophilisate was submitted for Fast Atom Bombardment Mass Spectrometry (FAB-MS). A second sample was submitted for amino acid sequence analysis.

### EXAMPLE 4. PREPARATION OF p-METHYL HIPPUROYL pGRF(2-76)OH.

### Part A. Synthesis of p-methylbenzoyl glycine.

Approximately 7.5 g of glycine was dissolved in a solution comprised of 105 ml of 2 N NaOH and 50 ml of dioxane with mechanical stirring at 0-5°C. Approximately 14.9 ml of p-toluoyl chloride was was diluted to a volume of 50 ml with dioxane and added dropwise to the reaction over a period of 15 to 20 minutes. The reaction mixture was stirred overnight with warming to 25°C (i.e. room temperature).

The reaction was basified with aqueous NaOH and extracted with diethyl ether. The aqueous phase was acidified to pH3.0 with 6N HCl. The aqueous phase was extracted with EtOAc, the EtOAc was washed with H₂O and dried over MgSO₄, filtered, and the filtrate concentrated in vacuo to near dryness. The solids were suspended in Et₂O, filtered and dried in vacuo to produce 14.25 g of what was later demonstrated to be p-methyl hippuric acid. The identity of the product was confirmed by melting point and elemental combustion analysis which compared favorably with the theoretical values for p-methyl hippuric acid. The melting point of the compound produced above was 151-154°C. The elemental combustion analysis data is given in Table 1 below.

### Part B. Synthesis of p-methyl hippuroyl pGRF (2-76) OH.

Approximately 1.65 g of p-methylhippuric acid, prepared in substantial accordance with the teachings of Example 4 Part A above, was dissolved in 15 ml of dimethylformamide (DMF) with stirring and cooling in an ice bath. Approximately 1 g of N-hydroxy succinimide was added to the solution followed by 1.9 g of DCC. The reaction was stirred overnight with warming to room temperature (25°C).

The DCU precipitate was isolated by filtration and the filtrate was concentrated in vacuo. The residual oil was diluted with Et₂O and some crystallization occurred. The solids were isolated by filtration, washed with Et₂O, and dried in vacuo to yield 3.28 g of product having a melting point of 167-170°C. The material was subjected to elemental combustion analysis the results of which compared well with the theoretical values expected for the succinimidyl ester of p-methyl hippuric acid (C₁₄H₁₄N₂0₅).

Approximately 72 mg of pGRF(2-76)OH prepared in substantial accordance with the teaching of Example 2.A. above, was dissolved in 3 ml of 0.1 M Tris-HCl, pH7.8, 30% propanol with stirring at room temperature. To the reaction 57 mg of the succinimidyl ester of p-methyl hippuric acid was added and the reaction stirred at room temperature while removing 5 µl samples at 15 min, 1 hour and 2 hours after introduction of the succinimidyl N-(p-methyl) hippuric acid to follow the progress of the reaction. Each of these samples was diluted to 500ml with 0.1% TFA and injected onto a 0.46 x 15 cm Vydac C18 column for RP-HPLC analysis and comparison with the starting material.

After 2.5 hours the reaction mixture was acidified with approximately 1 ml of glacial HOAc and applied to a 2.2 x 28.5 cm Sephadex G-10 column. The column was eluted with 10% HOAc and 7.5 ml fractions were collected. The ABS₂₈₀ of each fraction was determined to indicate the presence of polypeptidyl compounds. Fractions 7 through 10 indicating the presence of polypeptide were combined, frozen, and lyophilized to give 70mg of lyophilisate.

### EXAMPLE 5. PREPARATION OF p-CHLORO HIPPUROYL bGRF 52-77) OH.

Preparation of the p-chlorohippuroyl GRF derivative is accomplished in substantial accordance with the teaching of Example 4 above except that p-toluoyl chloride is replaced with p-chlorobenzoyl chloride in Part A and the bGRF(2-77)OH GRF PEPTIDE is prepared in substantial accordance with the teaching of Example 1 (solid phase).

### EXAMPLE 6. PREPARATION OF p-NITRO HIPPUROYL bGRF(2-77) OH.

Preparation of the p-nitrohippuroyl GRF derivative is accomplished in substantial accordance with the teaching of Example 4 above except that p-toluoyl chloride is replaced with p-nitrobenzoyl chloride in Part A and the bGRF(2-77)OH GRF PEPTIDE is prepared in substantial accordance with the teaching of Example 1 (solid phase).

### EXAMPLE 7. PREPARATION OF p-HYDROXY HIPPUROYL bGRF(2-77) OH.

Preparation of the p-hydroxyhippuroyl GRF derivative is accomplished in substantial accordance with the teaching of Example 4 above except that p-toluoyl chloride is replaced with p-hydroxybenzoyl chloride in Part A and the bGRF(2-77)OH GRF PEPTIDE is prepared in substantial accordance with the teaching of Example 1 (solid phase).

### EXAMPLE 8. PREPARATION OF p-FLUORO HIPPUROYL bGRF (2-77) OH.

Preparation of the p-fluorohippuroyl GRF derivative is accomplished in substantial accordance with the teaching of Example 4 above except that p-toluoyl chloride is replaced with p-fluorobenzoyl chloride in Part A and the bGRF(2-77)OH GRF PEPTIDE is prepared in substantial accordance with the teaching of Example 1 (solid phase).

### EXAMPLE 9. PREPARATION OF N-PHENYLMALONAMINDOYL-1-bGRF(2-77) OH.

Approximately 9.1 ml (0.1 mole) aniline was dissolved in 100 ml of CH₂Cl₂ with mechanical stirring at 25°C. To the solution was added 15.1 g K₂CO₃ followed by the dropwise addition of 14 ml of ethyl malonyl chloride in 20 ml of CH₂Cl₂. The reaction was stirred for 3-4 hours at 25°C and allowed to proceed for approximately 72 hours.

The reaction was diluted with approximately 500 ml of H₂O/CH₂Cl. The mixture was shaken and the CH₂Cl₂ phase was separated, washed first with aqueous HCl followed by H₂O and dried over MgSO₄, filtered and the filtrate evaporated to an oil of 14.5 g.

Approximately 14 g of ethyl N-phenylmalonamide prepared above was dissolved in 25 ml of dioxane and 75 ml of 1 N NaOH. The reaction was stirred at 25°C for approxmately 3 hours.

The reaction was extracted with Et₂O while still basic. The aqueous phase was acidified with 6N HCl and extracted with EtOAc and dried over MgSO₄. The EtOAC filtrate was evaporated to a solid. The solid was triturated in Et₂O, filtered and dried in vacuo to give 5.1 g of product. The product was analyzed by elemental combustion analysis and ¹H-NMR. The results indicated the product to be N-phenyl malonamide acid or N-phenylmalonic acid monoamide.

The N-phenyl malonamide acid was coupled to the bGRF(2-77)-resin via an HOBT ester.

### EXAMPLE 10. PREPARATION OF N-PHENYLMALONAMINDOYL-1-pGRF(2-77) OH.

3.58 g. of N-phenylmalonamide acid prepared in substantial accordance with the teaching of Example 9 above was dissolved in approximately 20 ml of DMF with stirring and cooled in an ice bath. To the solution was added 2.5 g of N-hydroxysuccinimide followed by 4.5 g dicyclohexyl carbodiimide (DCC). The reaction was stirred overnight with warming to room temperature.

The DCU was filtered and the DMF filtrate concentrated in vacuo. The residue was diluted with Et₂O. A precipitate formed which was filtered and dried in vacuo to give 3.79 grams of the succidimidyl ester N-phenylmalonamide acid.

Approximately 72 mg of pGRF(2-76)OH prepared in substantial accordance with the teaching of Example 2.A. above, was dissolved in 3 ml of 0.1 M Tris-HCl, pH7.8, 30% propanol with stirring at room temperature. To the reaction 57 mg of the succidimidyl ester of N-phenylmalonamide acid prepared above was added and the reaction stirred at room temperature while removing 5 µl samples at 15 min, 1 hour and 2 hours after introduction of the succidimidyl ester N-phenylmalonamide acid to follow the progress of the reaction. Each of these samples was diluted to 500µl with 0.1% TFA (.15-.2 mg/ml) and injected onto a 0.46 x 15 cm Vydac C18 column for RP-HPLC analysis and comparison with the starting material.

After 2.5 hours the reaction mixture was acidified with approximately 1 ml of glacial HOAc and applied to a 2.2 x 28.5 cm Sephadex G-10 column. The column was eluted with 10% HOAc and 7.5 ml fractions were collected. The ABS₂₈₀ of each fraction was determined to indicate the presence of polypeptidyl compounds. Fractions 7 through 10 indicating the presence of polypeptide were combined, frozen, and lyophilized to give 70 mg of lyophilisate.

### EXAMPLE 11. FORMULATION p-METHYL HIPPUROYL pGRF (2-76) OH

A carrier solution was prepared by dissolving 1.38 g of Na2HP04 and 1.0g PSA with pyrogen free H2O to a final volume of 1 litre, pH approximately 5.1. 27.4 mg The p-methyl hippuroyl pGRF (2-76)OH prepared in substantial accordance with the teaching of Example 4.B. above was added to 64 ml of the carrier solution to achieve a final concentration of 360 milliequivalents per milliliter.

### EXAMPLE 12. IN VIVO EFFECTS OF p-METHYL HIPPUROYL pGRF (2-76) OH WHEN INJECTED SUBCUTANEOUSLY OR INTRAVENOUSLY 3 TIMES DAILY IN BARROWS

Twenty crossbred barrows, averaging approximately 72 kg, with an indwelling catheter which had been surgically inserted into the femoral vein, were used to determine the effects of p-methyl hippuroyl pGRF (2-76)OH. A formulation of the p-methyl hippuroyl pGRF (2-76)OH was prepared in substantial accordance with the teaching of Example 11 above and injected 3 times daily at a rate of 10.0 µg/kg/inj., either intravenously (I.V.) or subcutaneously (S.Q.). The barrows were fed 2000 g of an 18.5% crude protein corn-soy diet per day, equally divided between 2 feedings at 8:00 a.m. and 4:00 p.m. The barrows were injected at 8:00 a.m., 4:00 p.m., and 12:00 p.m. Urinary urea nitrogen excretion data were collected during 1 5day control period, 4 3-day treatment periods, and 2 3-day periods after treatments were stopped. Thirty blood serum samples were collected during a 24-hour period on the 12th day of treatment. The serum samples were assayed for GH, BUN, glucose, insulin, FFA, and triglycerides. The means of the results are shown in Table 4 below.

**TABLE 4**

| TRT. | LEVEL | UREA | GH | BUN | GLUCOSE | FFA | TRIG. | INSULIN |
|---|---|---|---|---|---|---|---|---|
| | µg/kg/d | g/day | ng/ml | mg/dl | mg/dl | µM/la | mg/dla | µu/ml |
| CONT. | IV O | 27.5^{a} | 2.5^{a} | 15.8^{a} | 95.4^{a} | 55.8^{a} | 23.8^{a} | 20.8^{a} |
| TRT. | IV 30 | 16.3^{b} | 9.9^{b} | 7.0^{b} | 132.7^{b} | 97.3^{b} | 35.3^{b} | 76.8^{b} |
| CONT. | SQ O | 28.O^{a} | 2.1^{a} | 15.5^{a} | 101.4^{a} | 55.9^{a} | 24.3^{a} | 22.8^{a} |
| TRT. | SQ 30 | 16.4^{b} | 8.9^{b} | 7.5^{b} | 147.3^{b} | 97.5^{b} | 32.5^{ab} | 112.9^{C} |
| TRT.= p-methyl hippuroyl pGRF (2-76)OH, injected 3 times daily via the respective routes of administration | | | | | | | | |
| CONT.= The control barrows were injected 3 times daily with the carrier solution (phosphate buffer) via the respective routes of administration. | | | | | | | | |
| (a, b) = Means within same column with same letters are not different P<.05, Student-Newman-Keuls. | | | | | | | | |
| GH = Growth Hormone level | | | | | | | | |

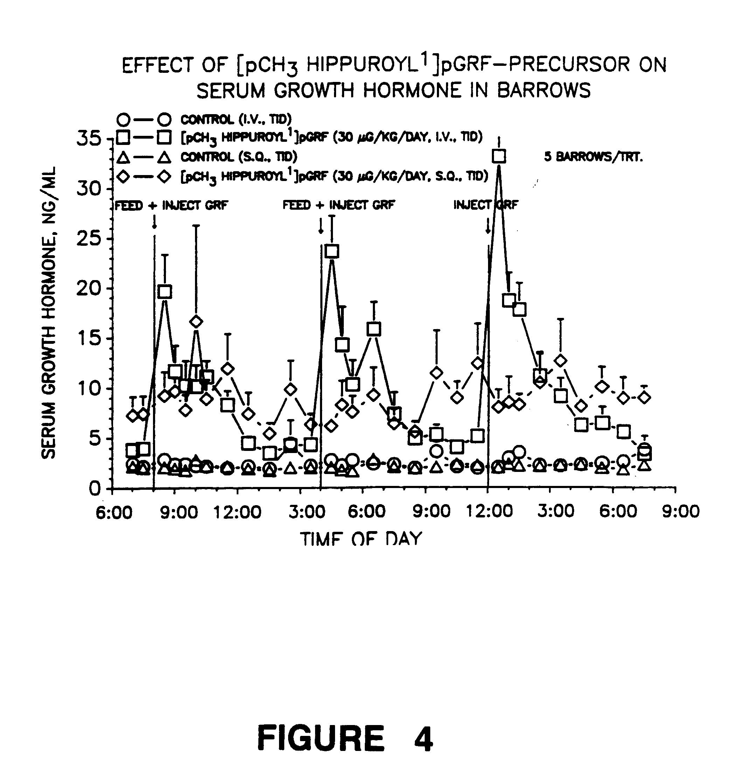

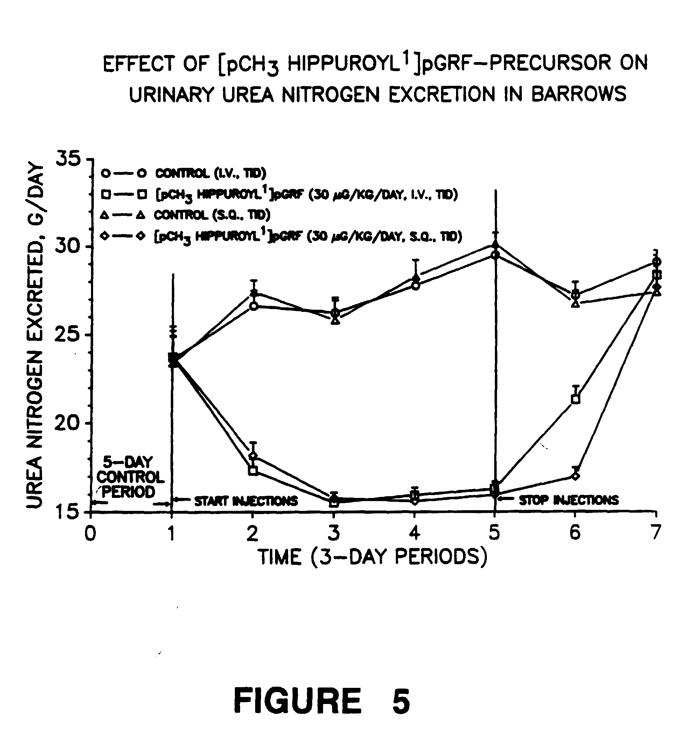

The data demonstrates that p-methyl hippuroyl pGRF (2-76)OH, when injected 3 times daily at a rate of 30.0 µg/kg/d, was very anabolic as indicated by a reduction in urinary urea nitrogen excretion (41%) and a reduction in serum BUN levels (54%). Injecting p-methyl hippuroyl pGRF (2-76)OH caused a 4-fold increase in serum GH levels (See Figure 4) which was accompanied by an increase in serum glucose, insulin, FFA, and triglycerides levels. There appeared to be no differences in the serum responses due to the method of administration, except serum insulin levels were increased more in the subcutaneously injected barrows than in the intravenously injected barrows. Also, the depression in urinary urea nitrogen excretion (See Figure 5) was prolonged for 2-3 days after treatments were stopped in the subcutanteously injected barrows.

## Claims (Claims for the following Contracting State(s): AT, BE, CH, LI, DE, DK, FR, GB, IT, LU, NL, PT, SE)

1. A compound of the formula: 
X1-Ala-Asp-Ala-Ile-Phe-Thr-Asn-A9-Tyr-Arg-A12-
Val-Leu-A15-Gln-Leu-Ser-Ala-Arg-A21-A22-Leu-Gln-
A25-Ile-A27-A28-Arg-Y-Z-T
 wherein:
A9 = Ser, Ala, Leu, Thr or Asn,
A12 = Arg or Lys,
A15 = Gly, Ala, Thr or Leu,
A21 = Arg or Lys,
A22 = Ala or Leu,
A25 = Asp or Glu,
A27 = Met, Ser, Arg, Leu, or Norleucine,
A28 = Ser or Asn,
Y = 0, or a peptide of from 1 to 15 amino acids,
Z = 0 or a peptide of from 1 to 32 amino acids,
T= a carboxyl terminal group represented by the formula -COCRₐ, -CRₐO, -CONHNHRₐ, -CON(Rₐ)(R_{b}) or CH₂ORₐ wherein Rₐ and R_{b} independently are lower alkyl, hydrogen, a Hse(lactone), HseOH or HseN(R_{c})(R_{d}) wherein R_{c} and R_{d} independently are hydrogen or lower alkyl,
X1 is an acyl group represented by the formula 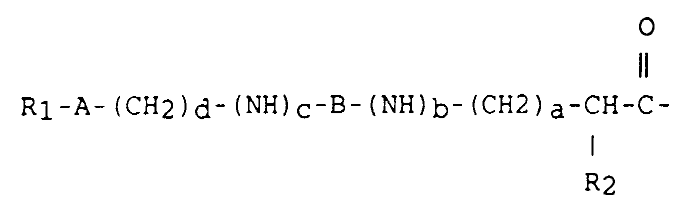
wherein
R₁ = p-methylphenyl,
a = zero,
b = 1,
c = zero,
d = zero,
A = zero,
B = carbonyl;
R₂ = H,
and the pharmaceutically acceptable non-toxic salts thereof.

2. The compound of Claim 1 wherein A9=Asn, A12=Arg, A15=Thr, A21=Arg, A22=Leu, A25=Asp, A27=Leu and A28=Ser.

3. The ccompound of any of Claim 2 wherein Y comprises a peptide of the amino acid sequence: Gln-Gln-Gly-Glu-Arg-Asn-Gln-Glu-Gln-Gly-Ala-Arg-Val-Arg-Leu, or a functional derivative thereof.

4. The compound of any of Claim 3 wherein Z comprises the amino acid sequence: Gly-Arg-Gln-Val-Asp-Ser-Leu-Trp-Ala-Asp-Gln-Arg-Gln-Leu-Ala-Leu-Glu-Ser-lle-Leu-Ala-Thr-Leu-Leu-Gln-Glu-His-Arg-Asn-Ser-Gln-Gly, or a functional derivative thereof.

5. The compound of Claim 1 wherein wherein A9=Ser, A12=Arg, A15=Thr, A21=Arg, A22=Leu, A25=Asp, and A28=Asn.

6. The compound of Claim 5 wherein Y comprises the amino acid sequence: Gln-Gln-Gly-Glu-Arg-Asn-Gln-Glu-Gln-Gly-Ala-Arg-Val-Arg-Leu, or a functional derivative thereof.

7. The compound of Claim 6 wherein Z comprises the amino acid sequence: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. The compound of Claim 1 wherein wherein A9 = Ser, A12 = Lys, A15 = Gly, A21= Lys, A22 = Leu, A25 = Asp, A27 = Met, and A28 = Ser.

9. The compound of Claim 8 wherein Y comprises the amino acid sequence: Glu-Gln-Gly-Glu-Ser-Asn-Gln-Glu-Arg-Gly-Ala-Arg-Ala-Arg-Leu or a functional derivative thereof.

10. The compound of Claim 9 wherein Z is zero.

11. A pharmaceutical formulation of comprising as an active ingredient a GRF analog as claimed in any one of claims 1 to 10, asociate with one or more pharmeceutically acceptable carriers, excipients, or diluents therefor.

12. A GRF analog as claimed in any of claims 1 to 11 for use in increasing the level of growth hormone.

13. A GRF analog as claimed in any of claims 1 to 10 for use in treating an organism suffering from hyperpituitary dwarfism.

14. A GRF analog as claimed in any of claims 1 to 10 for use in treating an organism suffering from osteoporosis.

15. A A GRF analog as claimed in any of claims 1 to 10 for use in increasing the lean muscle mass of an organism.

16. A method of preparing a GRF analog as claim in any of claims 1 to 10 which comprises,
a.) preparing a GRF PEPTIDE by chemical or recombinant DNA techniques, and
b.) chemically modifying the amino terminus.

## Claims (Claims for the following Contracting State(s): ES, GR)

1. A process for preparing a compound of the formula: 
X1-Ala-Asp-Ala-Ile-Phe-Thr-Asn-A9-Tyr-Arg-A12-
Val-Leu-A15-Gln-Leu-Ser-Ala-Arg-A21-A22-Leu-Gln-
A25-Ile-A27-A28-Arg-Y-Z-T
 wherein:
A9 = Ser, Ala, Leu, Thr or Asn,
A12 = Arg or Lys,
A15 = Gly, Ala, Thr or Leu,
A21 = Arg or Lys,
A22 = Ala or Leu,
A25 = Asp or Glu,
A27 = Met, Ser, Arg, Leu, or Norleucine,
A28 = Ser or Asn,
Y = 0, or a peptide of from 1 to 15 amino acids,
Z = 0 or a peptide of from 1 to 32 amino acids,
T= a carboxyl terminal group represented by the formula -COORₐ, -CRₐO, -CONHNHRₐ, -CON(Rₐ)(R_{b}) or CH₂ORₐ wherein Rₐ and R_{b} independently are lower alkyl, hydrogen, a Hse(lactone), HseOH or HseN(R_{c})(R_{d}) wherein R_{c} and R_{d} independently are hydrogen or lower alkyl,
X1 is an acyl group represented by the formula 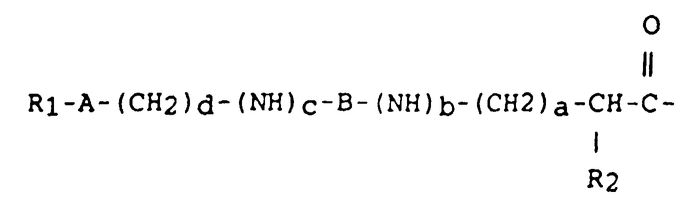
wherein
R₁ = p-methyl-phenyl,
a = zero,
b= 1,
c= zero,
d= zero,
A = zero,
B= carbonyl,
R₂ = H,
acetamido, or sulfamido, or a 5 or 6 member heterocycle containing one or more of N, O, or S, and the pharmaceutically acceptable non-toxic salts thereof.
which comprises,
a) preparing a des-Tyr1-GRF peptide by chemical or recombinant DNA techniques, and
b) chemically modifying the amino terminus.

2. A process according to Claim 1 wherein A9=Asn, A12=Arg, A15=Thr, A21=Arg, A22=Leu, A25=Asp, A27=Leu and A28=Ser, .

3. A process according to Claim 2 wherein Y comprises a peptide of the amino acid sequence: Gln-Gln-Gly-Glu-Arg-Asn-Gln-Glu-Gln-Gly-Ala-Arg-Val-Arg-Leu, or a functional derivative thereof.

4. A process according to Claim 3 wherein Z comprises the amino acid sequence: Gly-Arg-Gln-Val-Asp-Ser-Leu-Trp-Ala-Asp-Gln-Arg-Gln-Leu-Ala-Leu-Glu-Ser-lle-Leu-Ala-Thr-Leu-Leu-Gln-Glu-His-Arg-Asn-Ser-Gln-Gly, or a functional derivative thereof.

5. A process according to Claim 1 wherein wherein AS=Ser, A12=Arg, A15=Thr, A21=Arg, A22=Leu, A25=Asp, and A28=Asn.

6. A process according to Claim 5 wherein Y comprises the amino acid sequence: Gln-Gln-Gly-Glu-Arg-Asn-Gln-Glu-Gln-Gly-Ala-Arg-Val-Arg-Leu, or a functional derivative thereof.

7. A process according to Claim 6 wherein Z comprises the amino acid sequence: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. A process according to Claim 1 wherein wherein A9 = Ser, A12 = Lys, A15 = Gly, A21 = Lys, A22 = Leu, A25 = Asp, A27 = Met, and A28 = Ser.

9. A process according to Claim 8 wherein Y comprises the amino acid sequence: Glu-Gln-Gly-Glu-Ser-Asn-Gln-Glu-Arg-Gly-Ala-Arg-Ala-Arg-Leu or a functional derivative thereof.

10. A process according to Claim 9 wherein Z is zero.

11. A process for preparing a pharmaceutical formulation which comprises admixing a compound of the formula as defined in claim 1 with one or more pharmaceutically acceptable carriers, diluents, or excipients therefor.

## Patentansprüche (Patentansprüche für folgende(n) Vertragsstaat(en): AT, BE, CH, LI, DE, DK, FR, GB, IT, LU, NL, PT, SE)

1. Verbindung der Formel 
X1-Ala-Asp-Ala-Ile-Phe-Thr-Asn-A9-Tyr-Arg-A12-Val-Leu-A15-Gln-Leu-Ser-Ala-Arg-A21-A22-Leu-Gln-
A25-Ile-A27-A28-Arg-Y-Z-T
 worin
A9 für Ser, Ala, Leu, Thr oder Asn steht,
A12 für Arg oder Lys steht,
A15 für Gly, Ala, Thr oder Leu steht,
A21 für Arg oder Lys steht,
A22 für Ala oder Leu steht,
A25 für Asp oder Glu steht,
A27 für Met, Ser, Arg, Leu oder Norleucin steht,
A28 für Ser oder Asn steht,
Y für 0 oder ein Peptid von 1 bis 15 Aminosäuren steht,
Z für 0 oder ein Peptid von 1 bis 32 Aminosäuren steht,
T für eine terminale Carboxygruppe steht, die durch die Formel -COORₐ, CRₐO, -CONHNHRₐ, -CON(Rₐ)(R_{b}) oder CH₂ORₐ dargestellt wird, worin Rₐ und R_{b} unabhängig für niederes Alkyl, Wasserstoff, ein Hse(Lacton), Hse(OH) oder HseN(R_{c})(R_{d}) steht, worin R_{c} und R_{d} unabhängig für Wasserstoff oder niederes Alkyl stehen.
X1 für eine Acylgruppe der folgenden Formel steht 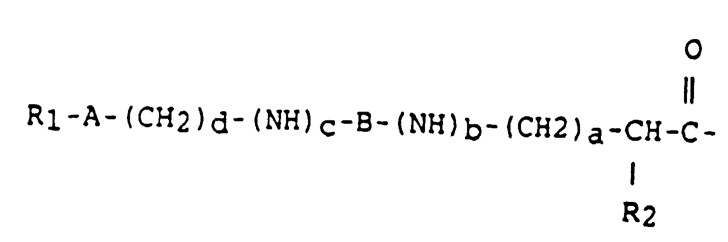
worin
R₁ für p-Methylphenyl steht,
a für Null steht,
b für 1 steht,
c für Null steht,
d für Null steht,
A für Null steht,
B für Carbonyl steht,
R₂ für H steht
und die pharmazeutisch annehmbaren nicht toxischen Salze hiervon.

2. Verbindung nach Anspruch 1, worin A9 für Asn, A12 für Arg, A15 für Thr, A21 für Arg, A22 für Leu, A25 für Asp, A27 für Leu und A28 für Ser steht.

3. Verbindung nach Anspruch 2, worin Y ein Peptid der folgenden Aminosäuresequenz umfaßt: Gln-Gln-Gly-Glu-Arg-Asn-Gln-Glu-Gln-Gly-Ala-Arg-Val-Arg-Leu oder ein funktionelles Derivat hiervon.

4. Verbindung nach Anspruch 3, worin Z die folgende Aminosäuresequenz umfaßt: Gly-Arg-Gln-Val-Asp-Ser-Leu-Trp-Ala-Asp-Gln-Arg-Gln-Leu-Ala-Leu-Glu-Ser-lle-Leu-Ala-Thr-Leu-Leu-Gln-Glu-His-Arg-Asn-Ser-Gln-Gly oder ein funktionelles Derivat hiervon.

5. Verbindung nach Anspruch 1, worin A9 für Ser, A12 für Arg, A15 für Thr, A21 für Arg, A22 für Leu, A25 für Asp und A28 für Asn steht.

6. Verbindung nach Anspruch 5, worin Y die folgende Aminosäuresequenz umfaßt: Gln-Gln-Gly-Glu-Arg-Asn-Gln-Glu-Gln-Gly-Ala-Arg-Val-Arg-Leu oder ein funktionelles Derivat hiervon.

7. Verbindung nach Anspruch 6, worin Z die folgende Aminosäuresequenz umfaßt: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. Verbindung nach Anspruch 1, worin A9 für Ser, A12 für Lys, A15 für Gly, A21 für Lys, A22 für Leu, A25 für Asp, A27 für Met und A28 für Ser steht.

9. Verbindung nach Anspruch 8, worin Y die folgende Aminosäuresequenz umfaßt: Glu-Gln-Gly-Glu-Ser-Asn-Gln-Glu-Arg-Gly-Ala-Arg-Ala-Arg-Leu oder ein funktionelles Derivat hiervon.

10. Verbindung nach Anspruch 9, worin Z für Null steht.

11. Pharmazeutische Formulierung, die als Wirkstoff ein GRF Analogon nach einem der Ansprüche 1 bis 10 zusammen mit einem oder mehreren pharmazeutisch annhembaren Trägern, Hilfsstoffen oder Verdünnungsmitteln hierfür enthält.

12. GRF Analogon nach einem der Ansprüche 1 bis 11 zur Verwendung bei der Erhöhung des Wachstumshormonspiegels.

13. GRF Analogon nach einem der Ansprüche 1 bis 10 zur Verwendung bei der Behandlung eines Organismus, der an Zwergwuchs durch Hyperpituitarismus leidet.

14. GRF Analogon nach einem der Ansprüche 1 bis 10 zur Verwendung bei der Behandlung eines Organismus, der an Osteoporose leidet.

15. GRF Analogon nach einem der Ansprüche 1 bis 10 zur Verwendung bei der Erhöhung der mageren Muskelmasse eines Organismus.

16. Verfahren zur Herstellung eines GRF Analogons nach einem der Ansprüche 1 bis 10, gekennzeichnet durch
a) Herstellung eines GRF Peptids durch chemische Techniken oder rekombinante DNA Techniken, und
b) Chemische Modifizierung des Aminoterminus.

## Patentansprüche (Patentansprüche für folgende(n) Vertragsstaat(en): ES, GR)

1. Verfahren zur Herstellung einer Verbindung der Formel 
X1-Ala-Asp-Ala-Ile-Phe-Thr-Asn-A9-Tyr-Arg-A12-Val-Leu-A15-Gln-Leu-Ser-Ala-Arg-A21-A22-Leu-Gln-
A25-Ile-A27-A28-Arg-Y-Z-T
 worin
A9 für Ser, Ala, Leu, Thr oder Asn steht,
A12 für Arg oder Lys steht,
A15 für Gly, Ala, Thr oder Leu steht,
A21 für Arg oder Lys steht,
A22 für Ala oder Leu steht,
A25 für Asp oder Glu steht,
A27 für Met, Ser, Arg, Leu oder Norleucin steht,
A28 für Ser oder Asn steht,
Y für 0 oder ein Peptid von 1 bis 15 Aminosäuren steht,
Z für 0 oder ein Peptid von 1 bis 32 Aminosäuren steht,
T für eine terminale Carboxygruppe steht, die durch die Formel -COORₐ, CRₐO, -CONHNHRₐ, -CON(Rₐ)(R_{b}) oder CH₂ORₐ dargestellt wird worin Rₐ und R_{b} unabhängig für niederes Alkyl, Wasserstoff, ein Hse(Lacton), Hse(OH) oder HseN(R_{c})(R_{d}) steht, worin R_{c} und R_{d} unabhängig für Wasserstoff oder niederes Alkyl stehen,
X1 für eine Acylgruppe der folgenden Formel steht 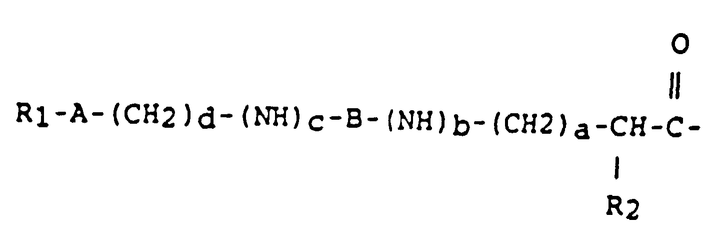
worin
R₁ für p-Methylphenyl steht,
a für Null steht,
b für 1 steht,
c für Null steht,
d für Null steht,
A für Null steht,
B für Carbonyl steht,
R₂ für H steht
und der pharmazeutisch annehmbaren nicht toxischen Salze hiervon. gekennzeichnet durch
a) Herstellung des Des-Tyrl-GRF Peptids durch chemische Techniken oder rekombinante DNA Techniken, und
b) Chemische Modifizierung des Aminoterminus.

2. Verfahren nach Anspruch 1, worin A9 für Asn, A12 für Arg, A15 für Thr, A21 für Arg, A22 für Leu, A25 für Asp, A27 für Leu und A28 für Ser steht.

3. Verfahren nach Anspruch 2, worin Y ein Peptid der folgenden Aminosäuresequenz umfaßt: Gln-Gln-Gly-Glu-Arg-Asn-Gln-Glu-Gln-Gly-Ala-Arg-Val-Arg-Leu oder ein funktionelles Derivat hiervon.

4. Verfahren nach Anspruch 3, worin Z die folgende Aminosäuresequenz umfaßt: Gly-Arg-Gln-Val-Asp-Ser-Leu-Trp-Ala-Asp-Gln-Arg-Gln-Leu-Ala-Leu-Glu-Ser-Ile-Leu-Ala-Thr-Leu-Leu-Gln-Glu-His-Arg-Asn-Ser-Gln-Gly oder ein funktionelles Derivat hiervon.

5. Verfahren nach Anspruch 1, worin A9 für Ser, A12 für Arg, A15 für Thr, A21 für Arg, A22 für Leu, A25 für Asp und A28 für Asn steht.

6. Verfahren nach Anspruch 5, worin Y die folgende Aminosäuresequenz umfaßt: Gln-Gln-Gly-Glu-Arg-Asn-Gln-Glu-Gln-Gly-Ala-Arg-Val-Arg-Leu oder ein funktionelles Derivat hiervon.

7. Verfahren nach Anspruch 6, worin Z die folgende Arninosäuresequenz umfaßt: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. Verfahren nach Anspruch 1, worin A9 für Ser, A12 für Lys, A15 für Gly, A21 für Lys, A22 für Leu, A25 für Asp, A27 für Met und A28 für Ser steht.

9. Verfahren nach Anspruch 8, worin Y die folgende Aminosäuresequenz umfaßt: Glu-Gln-Gly-Glu-Ser-Asn-Gln-Glu-Arg-Gly-Ala-Arg-Ala-Arg-Leu oder ein funktionelles Derivat hiervon.

10. Verfahren nach Anspruch 9, worin Z für Null steht.

11. Verfahren zur Herstellung einer pharmazeutischen Formulierung, gekennzeichnet durch Mischen einer Verbindung der in Anspruch 1 definierten Formel mit einem oder mehreren pharmazeutisch annhembaren Trägern. Verdünnungsmitteln oder Hilfsstoffen hierfür.

## Revendications (Revendications pour l'(les) Etat(s) contractant(s) suivant(s): AT, BE, CH, LI, DE, DK, FR, GB, IT, LU, NL, PT, SE)

1. Composé répondant à la formule: 
X1-Ala-Asp-Ala-Ile-Phe-Thr-Asn-A9-Tyr-Arg-A12-Val-Leu-A15-
Gln-Leu-Ser-Ala-Arg-A21-A22-Leu-Gln-A25-Ile-A27-A28-Arg-Y-Z-
T
 dans laquelle:
A9 = Ser, Ala, Leu, Thr ou Asn,
A12 = Arg ou Lys,
A15 = Gly, Ala, Thr ou Leu,
A21 = Arg ou Lys,
A22 = Ala ou Leu,
A25 = Asp ou Glu,
A27 = Met, Ser, Arg, Leu ou la norleucine,
A28 = Ser ou Asn,
Y = 0 ou un peptide contenant de 1 à 15 acides aminés,
Z = 0 ou un peptide contenant de 1 à 32 acides aminés,
T = un groupe terminal carboxyle répondant aux formules -COORₐ, -CRₐO, -CONHNHRₐ, -CON(Rₐ)(R_{b}) ou CH₂ORₐ où Rₐ et R_{b} représentent, indépendamment l'un de l'autre, un groupe alkyle inférieur, un atome d'hydrogène, un groupe Hse(lactone), un groupe HseOH ou un groupe HseN(R_{c})(R_{d}) où R_{c} et R_{d} représentent, indépendamment l'un de l'autre, un atome d'hydrogène ou un groupe alkyle inférieur,
X1 représente un groupe acyle répondant à la formule 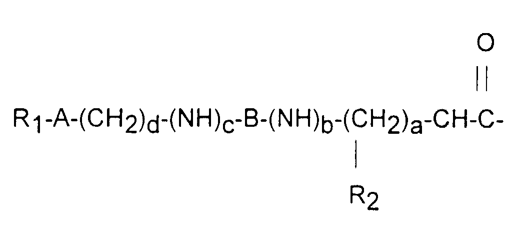
dans laquelle:
R₁ représente un groupe p-méthylphényle,
a = 0,
b = 1,
c = 0,
d = 0,
A = 0,
B représente un groupe carbonyle,
R₂ représente un atome d'hydrogène,
ainsi que ses sels non toxiques pharmaceutiquement acceptables.

2. Composé selon la revendication 1, dans lequel A9 = Asn, A12 = Arg, A15 = Thr, A21 = Arg, A22 = Leu, A25 = Asp, A27 = Leu et A28 = Ser.

3. Composé selon la revendication 2, dans lequel Y comprend un peptide contenant la séquence d'acides aminés: Gln-Gln-Gly-Glu-Arg-Asn-Gln-Glu-Gln-Gly-Ala-Arg-Val-Arg-Leu, ou un de ses dérivés fonctionnels.

4. Composé selon la revendication 3, dans lequel Z comprend la séquence d'acides aminés: Gly-Arg-Gln-Val-Asp-Ser-Leu-Trp-Ala-Asp-Gln-Arg-Gln-Leu-Ala-Leu-Glu-Ser-lle-Leu-Ala-Thr-Leu-Leu-Gln-Glu-His-Arg-Asn-Ser-Gln-Gly, ou un de ses dérivés fonctionnels.

5. Composé selon la revendication 1, dans lequel A9 = Ser, A12 = Arg, A15 = Thr, A21 = Arg, A22 = Leu, A25 = Asp et A28 = Asn.

6. Composé selon la revendication 5, dans lequel Y comprend la séquence d'acides aminés: Gln-Gln-Gly-Glu-Arg-Asn-Gln-Glu-Gln-Gly-Ala-Arg-Val-Arg-Leu, ou un de ses dérivés fonctionnels.

7. Composé selon la revendication 6, dans lequel Z comprend la séquence d'acides aminés: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. Composé selon la revendication 1, dans lequel A9 = Ser, A12 = Lys, A15 = Gly, A21 = Lys, A22 = Leu, A25 = Asp, A27 = Met et A28 = Ser.

9. Composé selon la revendication 8, dans lequel Y comprend la séquence d'acides aminés: Glu-Gln-Gly-Glu-Ser-Asn-Gln-Glu-Arg-Gly-Ala-Arg-Ala-Arg-Leu, ou un de ses dérivés fonctionnels.

10. Composé selon la revendication 9, dans lequel Z est égal à zéro.

11. Formulation pharmaceutique comprenant, à titre d'ingrédient actif, un analogue du GRF selon l'une quelconque des revendications 1 à 10, en association avec un ou plusieurs supports, excipients ou diluants pharmaceutiquement acceptables pour l'ingrédient actif.

12. Analogue du GRF selon l'une quelconque des revendications 1 à 11, à utiliser pour augmenter la concentration de l'hormone de croissance.

13. Analogue du GRF selon l'une quelconque des revendications 1 à 10, à utiliser dans le traitement d'un organisme souffrant de nanisme hypophysaire.

14. Analogue du GRF selon l'une quelconque des revendications 1 à 10, à utiliser dans le traitement d'un organisme souffrant d'ostéoporose.

15. Analogue du GRF selon l'une quelconque des revendications 1 à 10, à utiliser pour augmenter la masse des muscles pauvres en graisses d'un organisme.

16. Procédé pour préparer un analogue du GRF selon l'une quelconque des revendications 1 à 10, qui comprend le fait de
a.) préparer un PEPTIDE du GRF via des techniques chimiques ou d'ADN recombinant, et
b.) modifier par voie chimique l'extrémité terminale amino.

## Revendications (Revendications pour l'(les) Etat(s) contractant(s) suivant(s): ES, GR)

1. Procédé pour préparer un composé répondant à la formule: 
X1-Ala-Asp-Ala-Ile-Phe-Thr-Asn-A9-Tyr-Arg-A12-Val-Leu-A15-
Gln-Leu-Ser-Ala-Arg-A21-A22-Leu-Gln-A25-Ile-A27-A28-Arg-Y-Z-
T
 dans laquelle:
A9 = Ser, Ala, Leu, Thr ou Asn,
A12 = Arg ou Lys,
A15 = Gly, Ala, Thr ou Leu,
A21 = Arg ou Lys,
A22 = Ala ou Leu,
A25 = Asp ou Glu,
A27 = Met, Ser, Arg, Leu ou la norleucine,
A28 = Ser ou Asn,
Y = 0 ou un peptide contenant de 1 à 15 acides aminés,
Z = 0 ou un peptide contenant de 1 à 32 acides aminés,
T = un groupe terminal carboxyle répondant aux formules -COORₐ, -CRₐO, -CONHNHRₐ, -CON(Rₐ)(R_{b}) ou CH₂ORₐ où Rₐ et Rb représentent, indépendamment l'un de l'autre, un groupe alkyle inférieur, un atome d'hydrogène, un groupe Hse(lactone), un groupe HseOH ou un groupe HseN(R_{c})(R_{d}) où R_{c} et R_{d} représentent, indépendamment l'un de l'autre, un atome d'hydrogène ou un groupe alkyle inférieur,
X1 représente un groupe acyle répondant à la formule 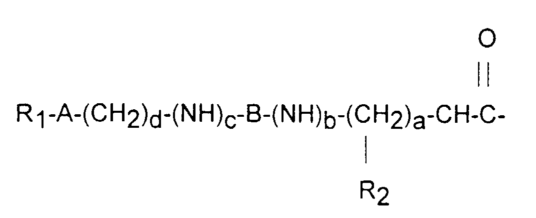
dans laquelle:
R₁ représente un groupe p-méthylphényle,
a = 0,
b = 1,
c = 0,
d = 0,
A = 0,
B représente un groupe carbonyle,
R₂ représente un atome d'hydrogène, un groupe acétamido ou un groupe sulfamido ou encore un composé hétérocyclique penta- ou hexagonal, contenant un ou plusieurs atomes d'azote, d'oxygène ou de soufre,
et d'un de ses sels non toxiques pharmaceutiquement acceptables, qui comprend le fait de :
a.) préparer un PEPTIDE du GRF via des techniques chimiques ou d'ADN recombinant, et
b.) modifier par voie chimique l'extrémité terminale amino.

2. Procédé selon la revendication 1, dans lequel A9 = Asn, A12 = Arg, A15 = Thr, A21 = Arg, A22 = Leu, A25 = Asp, A27 = Leu et A28 = Ser.

3. Procédé selon la revendication 2, dans lequel Y comprend un peptide contenant la séquence d'acides aminés: Gln-Gln-Gly-Glu-Arg-Asn-Gln-Glu-Gln-Gly-Ala-Arg-Val-Arg-Leu, ou un de ses dérivés fonctionnels.

4. Procédé selon la revendication 3, dans lequel Z comprend la séquence d'acides aminés: Gly-Arg-Gln-Val-Asp-Ser-Leu-Trp-Ala-Asp-Gln-Arg-Gln-Leu-Ala-Leu-Glu-Ser-lle-Leu-Ala-Thr-Leu-Leu-Gln-Glu-His-Arg-Asn-Ser-Gln-Gly, ou un de ses dérivés fonctionnels.

5. Procédé selon la revendication 1, dans lequel A9 = Ser, A12 = Arg, A15 = Thr, A21 = Arg, A22 = Leu, A25 = Asp et A28 = Asn.

6. Procédé selon la revendication 5, dans lequel Y comprend la séquence d'acides aminés: Gln-Gln-Gly-Glu-Arg-Asn-Gln-Glu-Gln-Gly-Ala-Arg-Val-Arg-Leu, ou un de ses dérivés fonctionnels.

7. Procédé selon la revendication 6, dans lequel Z comprend la séquence d'acides aminés: Gly-Arg-Gln-Val-Asp-Gly-Val-Trp-Thr-Asp-Gln-Gln-Gln-Leu-Ala-Leu-Glu-Ser-Thr-Leu-Val-Ser-Leu-Leu-Gln-Glu-Arg-Arg-Asn-Ser-Gln-Gly.

8. Procédé selon la revendication 1, dans lequel A9 = Ser, A12 = Lys, A15 = Gly, A21 = Lys, A22 = Leu, A25 = Asp, A27 = Met et A28 = Ser.

9. Procédé selon la revendication 8, dans lequel Y comprend la séquence d'acides aminés: Glu-Gln-Gly-Glu-Ser-Asn-Gln-Glu-Arg-Gly-Ala-Arg-Ala-Arg-Leu, ou un de ses dérivés fonctionnels.

10. Procédé selon la revendication 9, dans lequel Z est égal à zéro.

11. Procédé pour préparer une formulation pharmaceutique qui comprend le fait de mélanger un composé répondant à la formule telle que définie à la revendication 1 avec un ou plusieurs supports, diluants ou excipients pharmaceutiquement acceptables pour le composé.
